# EUROPEAN PATENT APPLICATION

(11) **EP 3 586 829 A1**
(43) Date of publication of application: **01.01.2020**
(21) Application number: 19191339.1
(22) Date of filing: 22.01.2013
(51) Int. Cl.: A61K 9/16, A61K 9/14, A61K 39/395, A61K 38/28, A61P 17/00, A61P 37/00, A61K 39/39, A61K 39/00

(54) **INULIN AND INULIN ACETATE FORMULATIONS**

(30) Priority: 20.01.2012 US 201261589126 P
(62) Divisional of application: 13738575.3
(71) Applicant: South Dakota State University, Brookings, SD 57006 (US)
(72) Inventor: TUMMALA, Hemachand, Brookings, SD South Dakota SD 57006 (US); KUMAR, Sunny, Brookings, SD South Dakota SD 57006 (US)
(74) Representative: advotec.

(57) **Abstract**

The disclosure provides compositions that include microparticles or nanoparticles of beta inulin or inulin acetate and an active agent, where the active agent is contained within individual microparticles or nanoparticles. The active agent can be, for example, a vaccinating antigen, an antigenic peptide sequence, or an immunoglobulin. The compositions can be incorporated into various formulations for administration to a subject such as a human or animal. The invention further provides methods of using the compositions and formulations, including methods of stimulating an immune response in a subject, or enhancing an immune response in a subject, for the purposes of treating, preventing, or inhibiting an infectious disease, autoimmune disease, immunodeficiency disorder, neoplastic disease, degenerative disease, an aging disease, or a combination thereof.

## Description

### BACKGROUND OF THE INVENTION

### FIELD OF THE INVENTION

The present invention relates generally to vaccines and adjuvants, and more specifically to vaccines and adjuvants comprising β-inulin and β-inulin derivatives, wherein said β-inulin and β-inulin derivatives serve as a delivery devices as well as adjuvants, including nanoparticle and microparticle compositions comprised thereof and methods of treatment using said compositions.

### BACKGROUND INFORMATION

The goal of vaccination is to provide long-term protection against infection by generating a strong immune response to the administered antigen. Vaccines often require the addition of immune stimulatory agents called adjuvants to boost the potency and longevity of specific immune response to antigens. An ideal vaccine adjuvant should stimulate both humoral (Th2 type) and cellular (Thl type) immune responses against co-injected antigens. A humoral response is needed to clear extracellular pathogens. A cellular response is critical to eliminate intracellular pathogens.

Alum (aluminum salts), the only approved adjuvant for human use in the U.S., stimulates only the humoral immune response. Although some recombinant protein-based vaccines, including those for Hepatitis B and human papilloma virus, have been successfully developed to elicit protective antibody responses using only alum as an adjuvant, the next generation of recombinant vaccines, aimed at diseases such as cancer, malaria, herpes, influenza, tuberculosis, HIV and/or AIDS, will require not only very strong and long lasting antibody responses but also potent cell mediated immune responses.

Accordingly, there is a need for new compositions that can promote antibody responses in subjects. There is also a need for novel vaccine adjuvants, antigen delivery systems, and compositions that can promote both humoral and cellular immune responses.

### SUMMARY OF THE INVENTION

The present invention provides soluble or insoluble polysaccharide polymers that stimulate the immune system when delivered as microparticles or nanoparticles. The beta isoform of inulin (β-Inulin) and its semi-synthetic derivative Inulin Acetate (InAc) can function as novel vaccine adjuvants and antigen delivery systems when formulated as micro or nano particles. The particles may be used to stimulate an immune response in a subject for the purposes of preventing or inhibiting an infectious disease, an autoimmune disease, an immunodeficiency disorder, a neoplastic disease, a degenerative disease, or an aging disease.

In embodiments, a composition including microparticles or nanoparticles of β-inulin or inulin acetate and an active agent is disclosed, where the active agent may be contained within individual microparticles or nanoparticles. The active agent may include an antigen, an antigenic peptide or sequence thereof, an immunogen, an immunoglobulin, or a combination thereof. In a related aspect, the active agent may be a hapten or any agent against which an immune response is desired.

In embodiments, a composition including β-inulin or inulin acetate particles and an active agent is disclosed, where the active agent may be contained within the individual particles, or physically associated with the particles, of β-inulin or inulin acetate, where the composition, when administered to a human or an animal, is effective to stimulate an immune response against the active agent in the human or animal.

In one aspect, the vaccine composition comprises an antigen and an effective adjuvant amount of inulin acetate, where the degree of acetylation on the inulin acetate is about 0.1 % to 100%.

In another aspect, a composition includes microparticles or nanoparticles of β-inulin or an inulin derivative and an active agent, the active agent includes an antigen, an antigenic peptide sequence, or an immunoglobulin, and the composition includes an effective adjuvant amount of β-inulin or an inulin derivative, where the inulin derivative includes esterified inulin, etherified inulin, dialdehyde inulin, inulin carbamate, inulin carbonate, oxidized or reduced forms of inulin, complexation of inulin or its oxidized or reduced form with an additional active agent, cationic/anionic/non-ionic modifications of inulin, or inulin phosphates, in the form of particulate formulations where the antigen is encapsulated within the particles, coated or conjugated on to the particles or may include combinations thereof.

In embodiments, a method of stimulating an immune response in a subject, for the purposes of treating or inhibiting an infectious disease, autoimmune disease, immunodeficiency disorder, neoplastic disease, genetic disease, degenerative or ageing disease is disclosed, where the method includes administering to a subject in need thereof an effective amount of a composition described herein.

In embodiments, provides a method of enhancing an immune response in a subject, for the purposes of treating or inhibiting an infectious disease, autoimmune disease, immunodeficiency disorder, neoplastic disease, genetic disease, degenerative or aging disease is disclosed, where the method includes administering to a subject in need thereof an effective amount of a composition described herein.

In embodiments, a method of inducing an immunogenic response in a subject is disclosed including administering to said subject an effective amount of a composition described herein.

In another embodiment, a method to treat an infection is disclosed including administering to a subject in need thereof an effective amount of a composition described herein. The infection may be caused by a bacterium, mycoplasma, fungus, virus, protozoan, or other microbe, or a combination thereof.

In embodiments, a method to treat an infestation in an animal or a human is disclosed including administering to an animal or a human afflicted with an infestation an effective amount of a composition described herein. The infestation may be caused by a microbe, a worm, a parasite, or a combination thereof.

In embodiments, a method for producing antibodies or for stimulating immune cells is disclosed including immunizing an animal or a human with a formulation comprising a composition described herein, and collecting the antibodies or immune cells from the immunized animal or human. The antibodies may be, for example, monoclonal antibodies or polyclonal antibodies or functional fragments thereof (e.g., Fab, scFv, antibody conjugates or the like).

In embodiments, a method for producing antisera is disclosed including immunizing an animal or a human with a formulation having a composition described herein and collecting the antisera from the immunized animal or human, or from a product of the immunized animal or human (such as an egg of the animal). The antisera may contain antibodies, where the antibodies may be monoclonal antibodies or polyclonal antibodies. The animal may be a primate, dog, cat, cow, lamb, pig, hog, poultry, horse, mare, mule, jack, jenny, colt, calf, yearling, bull, ox, sheep, goat, llama, bison, buffalo, lamb, kid, shoat, hen, chicken, turkey, duck, goose, ostrich, fowl, rabbit, hare, guinea pig, hamster mouse, rat, rodents, fish, aquatic animal, or amphibian.

The composition may be administered by injection, inhalation, orally, rectally, vaginally, nasally, or topically. The active agent may be prepared in combination with a physiologically acceptable, non-toxic vehicle prior to encapsulation or association with the particles, or the particles may be combined with a physiologically acceptable, non-toxic vehicle after encapsulation or association with the active agent.

In one aspect, microparticles or nanoparticles described herein may be used as an adjuvant. In a related aspect, the particles of a composition described herein may be microparticles. The microparticles may have diameters of about 1 gm to about 30 µm, about 1.5 gm to about 25 µm, or about 2 µm to about 20 µm. The particles can also be nanoparticles. The nanoparticles may have diameters of about 10 nm to about 1000 nm, about 10 nm to about 950 nm, about 10 nm to 5 about 900 nm, about 50 nm to about 900 nm, about 100 nm to about 800 nm, about 10 nm to about 400 nm, about 400 nm to about 900 nm, or about 20 nm to about 750 nm.

In another aspect, the β-inulin or inulin acetate may have a molecular weight of about 4 kDa to about 25 kDa.

In one aspect, the composition may further include one or more immune modulators such as lymphokines, cytokines, thymocyte stimulators, monocyte or macrophage stimulators, endotoxins, pathogen associated molecular pattern (PAMS), ligands for pattern recognition receptors (PRRs), or a combination thereof.

In another aspect, the active agent may be an antigen, an antigenic peptide, or antigenic sequence, or an immunoglobulin. In a related aspect, the antigen may be, for example, DNA or RNA, or a biological component that includes DNA or RNA. In a further related aspect, the active agent may also include a lysate of a bacterial or a viral pathogen, cancer cell, or other biological component associated with a pathogen or a disease. For example, the biological component may be a peptide, a protein, a lipid, DNA, or a separate polysaccharide.

In one aspect, the composition may further include an effective amount of a second active agent, encapsulated or associated with the particles, or included in a formulation along with the particles.

In another aspect, the administration of a composition described herein may stimulate an immune response. The immune response may include Th1 (IgG-2a, cytotoxic T-cells) or Th2 (IgG-1, IgA) types of immune response, or a combination thereof. In a related aspect, the administration to an animal or human may reduce at least one of the symptoms caused by the active agent, or disease or condition associated with the active agent.

In one aspect, an immunological composition includes a composition described herein in combination with a pharmaceutically acceptable carrier, diluent, or excipient. In a related aspect, the immunological composition may be formulated as a liquid, semisolid, colloidal, or solid-dosage form. In a further related aspect, the carrier may be a liquid vehicle or the diluent can be a solid diluent, for example, for dry powder inhalation.

In embodiments, micro- and nano-particle formulations of β-Inulin or β-Inulin derivatives such as inulin acetate, and their use as immune stimulants are disclosed. In a related aspect, the formulations may have a burst release of less than about 30 wt.% of the encapsulated or physically associated molecules in the first 30 minutes after administration to a subject.

In embodiments, the formulation may have a burst release of less than about 20 wt.%, a burst release of less than about 15 wt.%, or a burst release of less than about 10 wt.%, in the first 30 minutes after administration to a subject. In a related aspect, release of > about 90 wt.% of an encapsulated material is > about 20 days.

In embodiments, the use of the compositions described herein for medical, veterinary, and zoonotic therapy or prevention of diseases is disclosed. In one aspect, the medical therapy may be preventing and treating cancer or other malignancies, including, but not limited to breast cancer, lung cancer, pancreatic cancer, prostate cancer, brain, or colon cancer, as well as lymphomas and leukemias, and cancers of the bone, blood, or lymphatic systems.

In embodiments, the use of composition as described herein for the manufacture of a medicament to treat a disease in a mammal, for example, cancer in a human is disclosed. In one aspect, the use of a composition as described herein for the manufacture of a medicament to treat a disease in a non-mammal species is disclosed. In a related aspect, medicaments may include a pharmaceutically acceptable diluent, excipient, or carrier.

In embodiments, a kit is disclosed including a composition comprising microparticles or nanoparticles of β-inulin or inulin acetate; optionally an active agent; a container; one or more buffers, instructions for associating an active agent with said composition; and a label.

### BRIEF DESCRIPTION OF THE DRAWINGS

The following drawings form part of the specification and are included to further demonstrate certain embodiments or various aspects of the disclosure. In some instances, embodiments of the disclosure may be best understood by referring to the accompanying drawings in combination with the detailed description presented herein.

The description and accompanying drawings may highlight a certain specific example, or a certain aspect of the disclosure. However, one skilled in the art will understand that portions of the example or aspect may be used in combination with other examples or aspects of the disclosure.

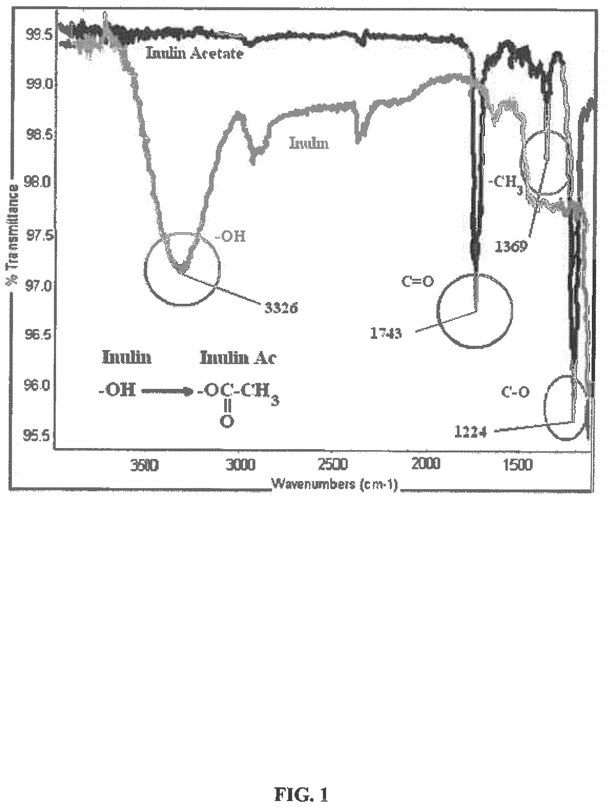

FIG. 1 shows a comparison of IR spectra of β-inulin and inulin acetate by FTIR spectroscopy.

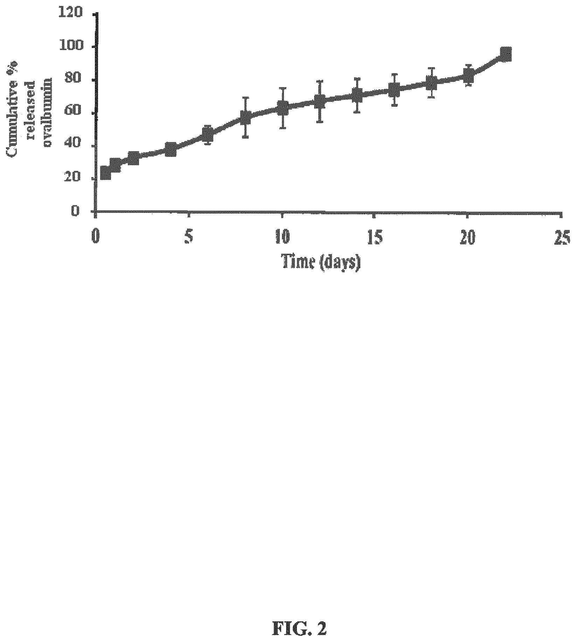

FIG. 2 shows an in-vitro release profile of ovalbumin from InAc microparticles. Release studies were performed with 10 mg of microparticles dispersed in 1 mL of 100 mM PBS at pH 7.4 with -100 rpm shaking at 37 °C. At different time intervals samples were taken and replaced with equal volume of fresh PBS. Ovalbumin concentration was measured by the bicinchoninic acid (BCA) protein assay (n=3).

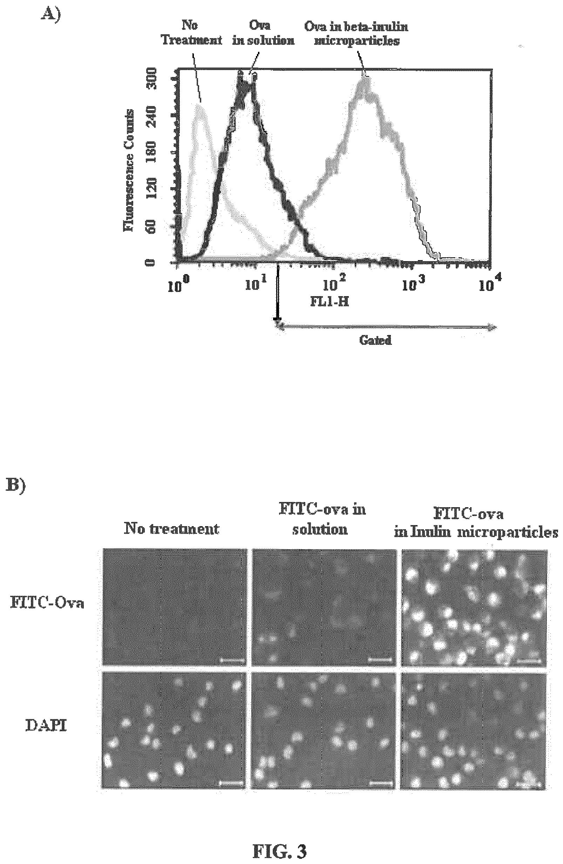

FIG. 3 shows the uptake of antigen (ova) by dendritic cells. (A) Flow Cytometry after incubation of DC2.4 cells with FITC ova either in solution or in inulin microparticles. (B) Same results as in 3A shown by fluorescence microscope, nucleus of DC2.4 cells was stained with DAPI.

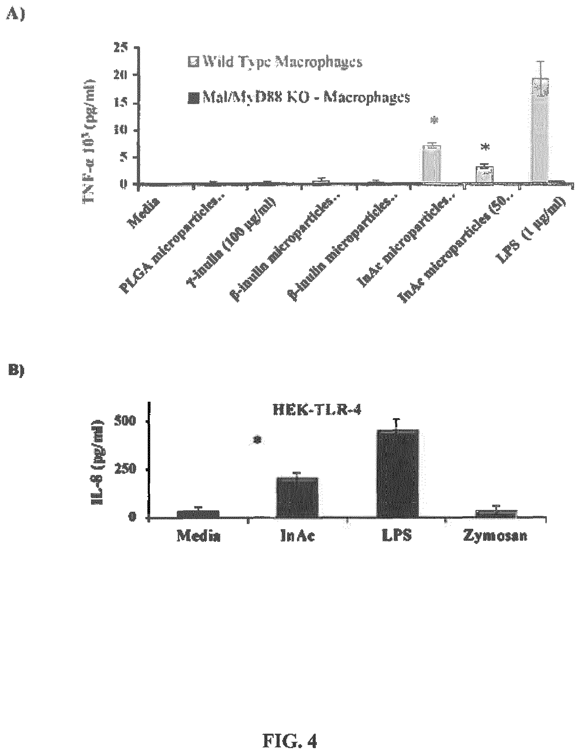

FIG. 4 shows (A) InAc activates macrophage through MyD88- or Mal-dependent TLRs and (B) InAc microparticles interact with TLR-4. * indicates that results are statistically significant (P < 0.05) using student t-test.

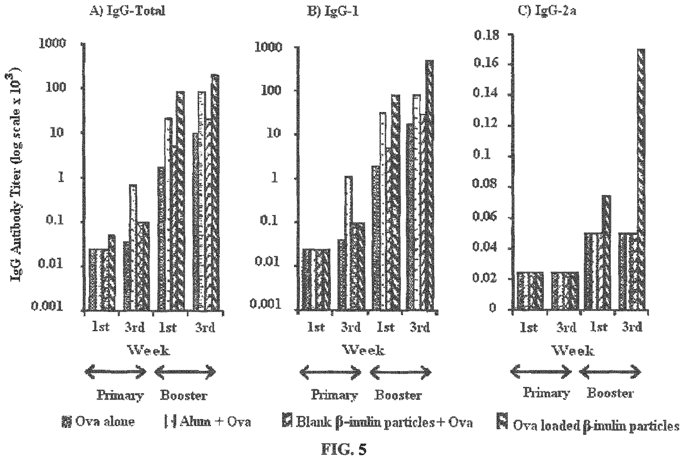

FIG. 5 shows ova-specific IgG-Total, IgG-1 and IgG-2a antibody titers in immunized mice serum. Mice (n = 4-5 per group) were injected intradermally with ova (100 µg) alone, ova with alum (200 µg), ova with blank β-inulin microparticles, or ova-loaded in β-inulin microparticles, on days 1 and 21 as primary and booster doses. Sera were collected at 1 and 3 weeks after immunizations for analysis of anti-ova antibody titers using indirect ELISA.

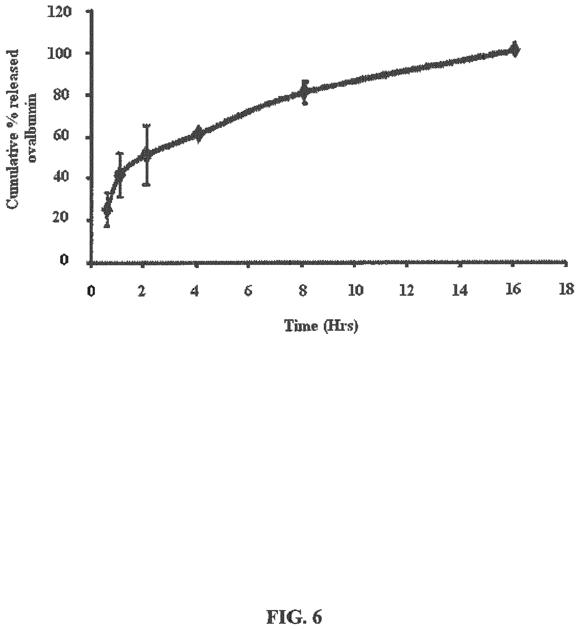

FIG. 6 shows a release profile of ovalbumin (ova) from β-inulin microparticles. Release studies were performed with 10 mg of microparticles dispersed in 1 mL of 100 mM PBS (phosphate buffer saline) at pH 7.4 with -100 rpm shaking at 37° C. At different time intervals samples were taken and replaced with equal volume of fresh PBS. FITC-Ova concentration was measured by fluorometric analysis (n=3).

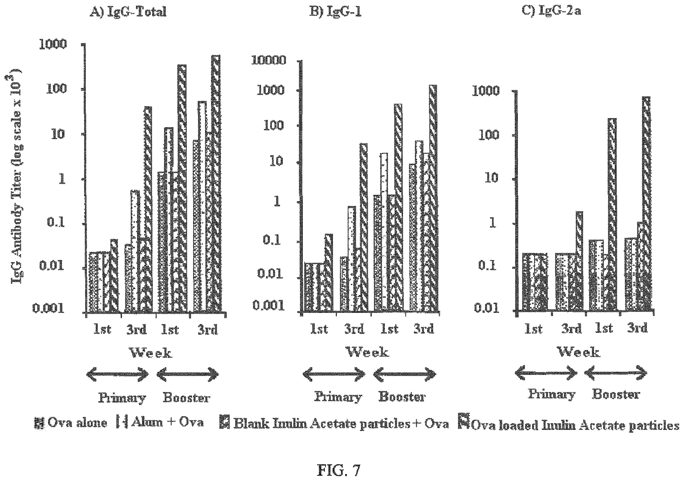

FIG. 7 shows ova-specific IgG-Total, IgG-1 and IgG-2a antibody titers in immunized mice serum. Mice (n = 4-5 per group) were injected intradermally with ova (100 µg) alone, ova with alum (200 µg), ova with blank InAc microparticles, or ova loaded in InAc microparticles, on 10 days 1 and 21 as primary and booster immunization. Sera were collected at 1 and 3 weeks after the primary and booster immunizations for analysis of IgG titers using indirect ELISA.

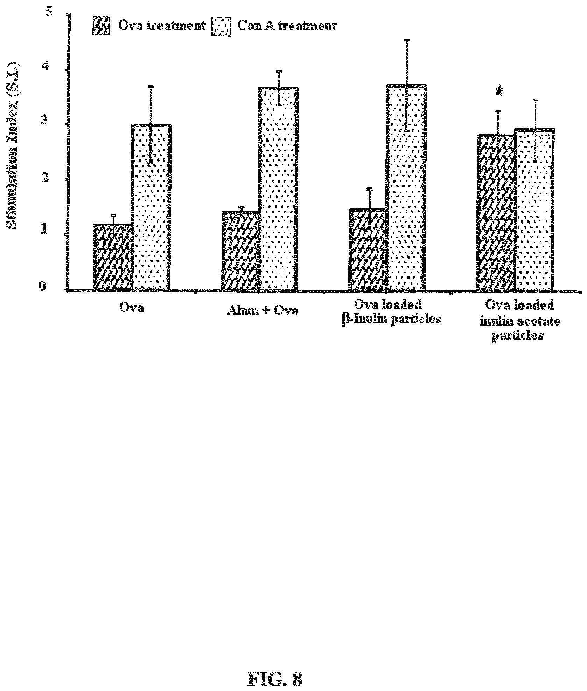

FIG. 8 shows in-vitro splenocyte proliferation in response to antigen (Ova). Splenocytes were prepared from mice immunized with ova alone, ova with Alum, or ova loaded in β-inulin or InAc microparticles and were cultured for 72 hours in the presence of Concanavalin A (ConA, 2.5µg/mL) or ova (100µg/mL) or RPMI 1640 media. Splenocyte proliferation was measured by the MTT assay and shown as a stimulation index (SI). SI = the absorbance value for antigen (ova) or mitogen (ConA) treated cultures divided by the absorbance value for non-stimulated cultures (RPMI treated). ConA is a positive control for proliferation. * indicates values are statistically significant compared to ova immunized group (P < 0.001) using the student t-test.

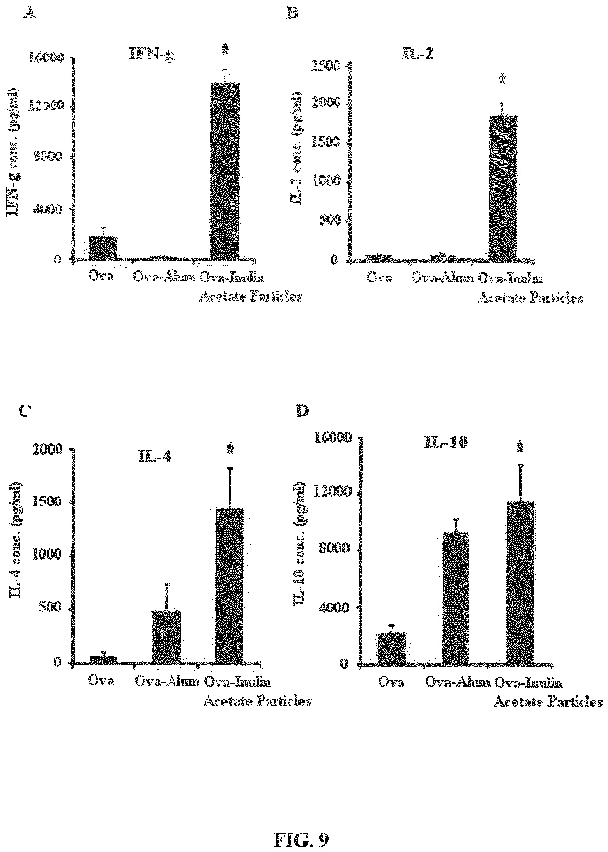

FIG. 9 shows the measurement of Th1 (IFN-g and IL-2) and Th2 (IL-4 and IL-10) cytokines. Splenocytes were prepared from mice immunized with ova alone, ova with Alum, or ova loaded InAc microparticles and were cultured for 72 hours in the presence of Ova (100µg/mL). After 72 hours, supernatant from different treatment groups were collected and concentration of different cytokines were measured using sandwich ELISA. * indicates the values are significantly higher compared to ova immunized group (P < 0.001) using the student t-test.

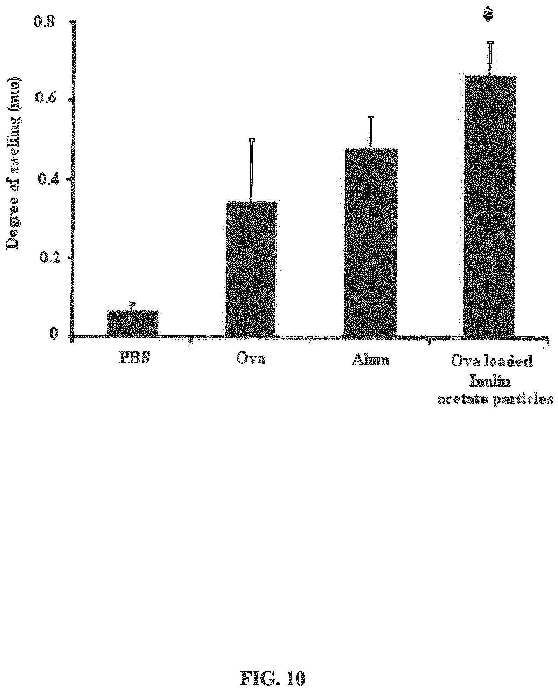

FIG. 10 shows DTH responses in immunized mice. Ova (5 µg) was injected in the left footpad and PBS in the right footpad of each immunized mice. The degree of footpad swelling was measured 24 hour after the Ova and PBS treatment. Data represents mean degree of swelling + standard deviation from 3-4 immunized mice of each group. * indicates values are statistically significant compared to the ova immunized group (P < 0.001) using the student t test.

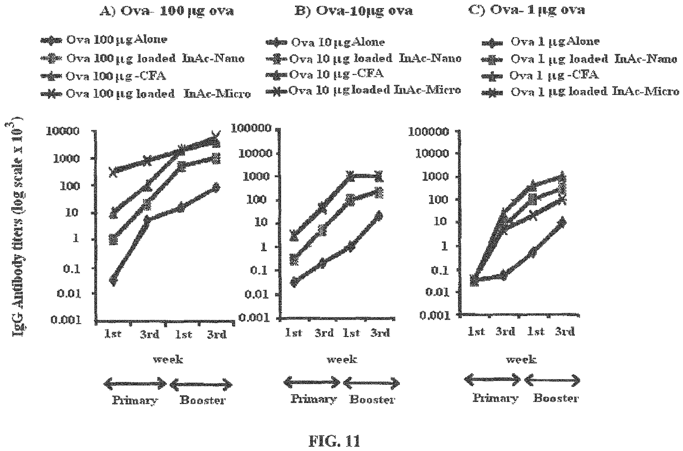

FIG. 11 shows the effect of size of inulin acetate particles on generation of ova-specific IgG-total titers in immunized mice serum. Mice (n = 4-5 per group) were injected subcutaneously with ova (100, 10 or 1 µg) alone or along with CFA (Complete Freund's Adjuvant) or loaded in InAc micro or nanoparticles on days 1 and 21 as primary and booster immunization. Sera were collected at 1 and 3 weeks after the primary and booster immunizations for analysis of IgG-total titers using indirect ELISA. CFA was used as a positive control (strongest adjuvant).

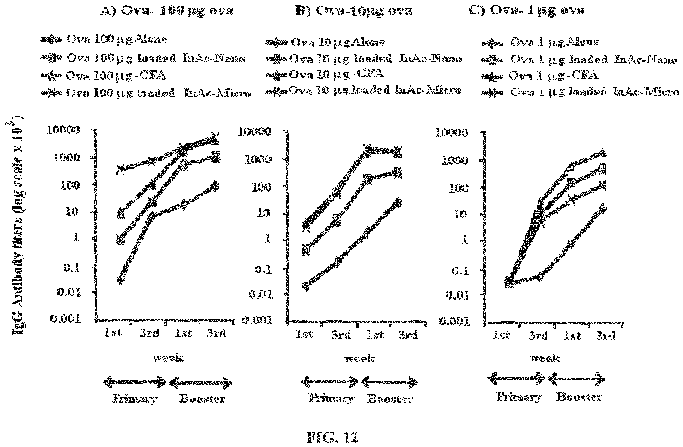

FIG. 12 shows the effect of size of inulin acetate particles on generation of serum ova-specific IgG-1 titers in immunized mice serum. Mice (n = 4-5 per group) were injected subcutaneously with ova (100, 10 or 1 µg) alone or along with CFA or loaded in InAc micro or nanoparticles on days 1 and 21. Sera were collected at 1 and 3 weeks after the primary and booster immunizations for analysis of IgG-1 titers using indirect ELISA.

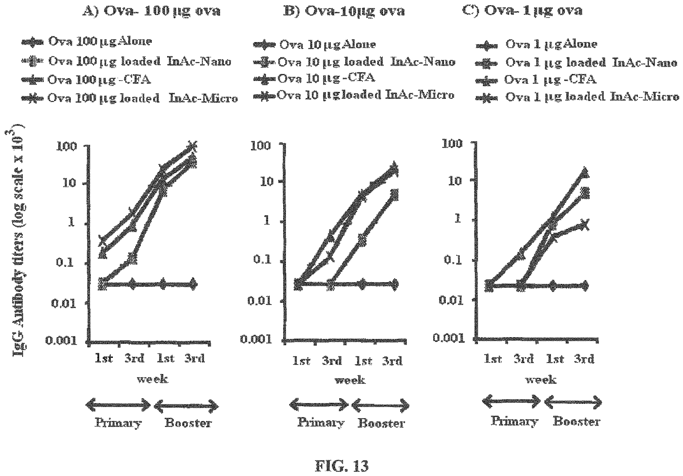

FIG. 13 shows the effect of size of inulin acetate particles on generation of ova-specific IgG-2a titers in immunized mice serum. Mice (n = 4-5 per group) were injected subcutaneously with ova (100, 10 or 1 µg) alone or along with CFA or loaded in InAc micro or nanoparticles on days 1 and 21. Sera were collected at 1 and 3 weeks after the primary and booster immunizations for analysis of IgG-2a titers using indirect ELISA.

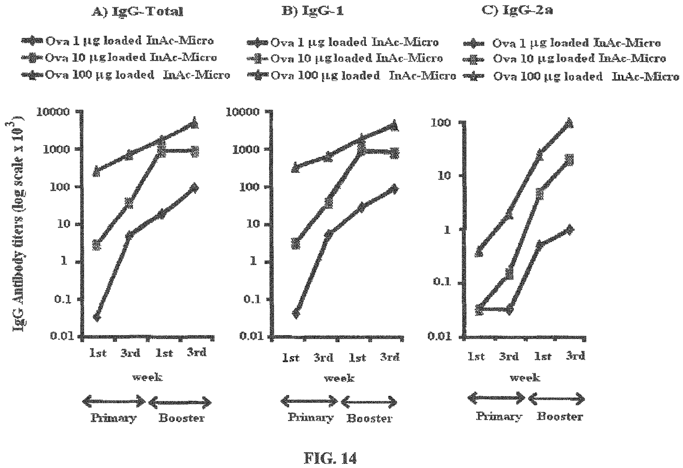

FIG. 14 shows the effect of amount of an antigen loaded in InAc microparticles on generation of ova-specific IgG titers in immunized mice serum. Mice (n = 4-5 per group) were injected subcutaneously with ova (100, 10 or 1 µg) alone or along with CFA or loaded in InAc microparticles on days 1 and 21. Sera were collected at 1 and 3 weeks after the primary and booster immunizations for analysis of IgG-total, IgG-1 and IgG-2a titers using indirect ELISA.

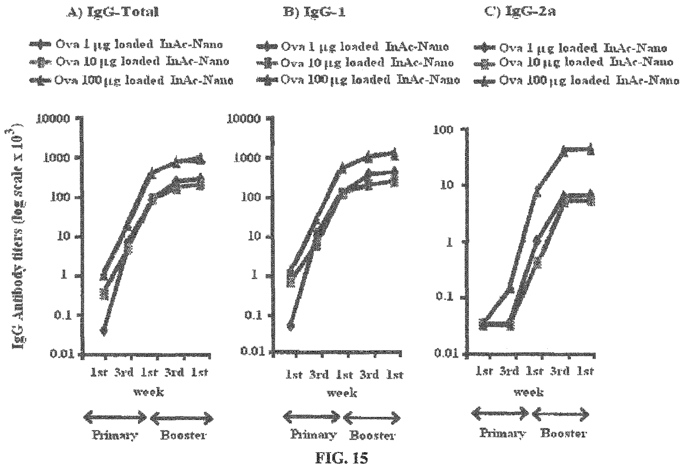

FIG. 15 shows the effect of the amount of an antigen loaded in InAc nanoparticles on generation of ova-specific IgG titers in immunized mice serum. Mice (n = 4-5 per group) were injected subcutaneously with ova (100, 10 or 1 µg) alone or along with CFA or loaded in InAc nanoparticles on days 1 and 21. Sera were collected at 1 and 3 weeks after the primary and booster immunizations for analysis IgG-total, IgG-1 and IgG-2a titers using indirect ELISA.

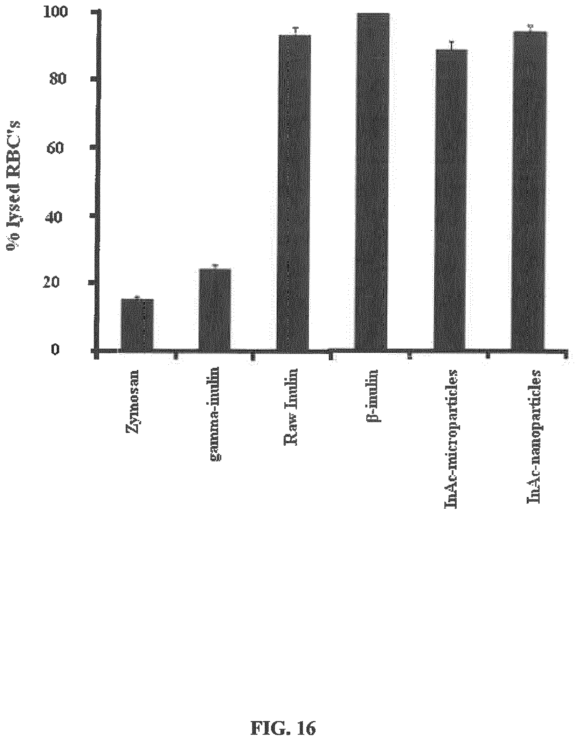

FIG. 16 shows results of an Alternate Pathway of Complement (APC) activation assay. Human serum causes the lysis of rabbit RBCs, which was analyzed by determining the OD values at 700 nm. Human serum was treated with various samples before incubating with RBCs for lysis. Percent RBC lysis was calculated by considering the RBC lysis with untreated human serum as 100 %. Zymosan (positive control) activates APC and hence inhibited the rabbit RBC's lysis.

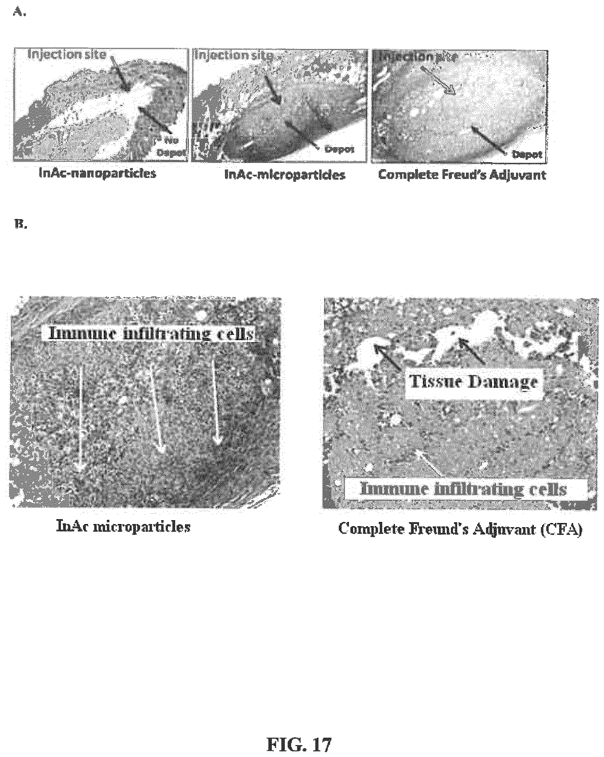

FIG. 17 shows an H&E staining of skin tissue at an adjuvant injection site. (A) shows three images from site of InAc-nanoparticle injection (left), InAc-microparticle injection (middle), and Complete Freud's adjuvant injection (CFA) (right) at low magnification and (B) shows two images at higher magnification, with InAc microparticles (left) and CFA (right).

### DETAILED DESCRIPTION OF THE INVENTION

### Definitions

As used herein, the recited terms have the following meanings. All other terms and phrases used in this specification have their ordinary meanings as one of skill in the art would understand. Such ordinary meanings may be obtained by reference to technical dictionaries, such as Hawley's Condensed Chemical Dictionary, 14th Edition, by R.J. Lewis, John Wiley & Sons, New York, N.Y., 2001.

References in the specification to "one embodiment", "an embodiment", etc., indicate that the embodiment described may include a particular aspect, feature, structure, moiety, or characteristic, but not every embodiment necessarily includes that aspect, feature, structure, moiety, or characteristic. Moreover, such phrases may, but do not necessarily, refer to the same embodiment referred to in other portions of the specification. Further, when a particular aspect, feature, structure, moiety, or characteristic is described in connection with an embodiment, it is within the knowledge of one skilled in the art to affect or connect such aspect, feature, structure, moiety, or characteristic with other embodiments, whether or not explicitly described.

The singular forms "a," "an," and "the" include plural reference unless the context clearly dictates otherwise. Thus, for example, a reference to "a compound" includes a plurality of such compounds, so that a compound X includes a plurality of compounds X. It is further noted that the claims may be drafted to exclude any optional element. As such, this statement is intended to serve as antecedent basis for the use of exclusive terminology, such as "solely," "only," and the like, in connection with the recitation of claim elements or use of a "negative" limitation.

The term "and/or" means any one of the items, any combination of the items, or all of the items with which this term is associated. The phrase "one or more" is readily understood by one of skill in the art, particularly when read in context of its usage. For example, one or more optional ingredients may be included in a particular formulation, thus one or more may refer to one to about four, or one to about five, or as many ingredients are desired in a particular formulation.

The term "about" may refer to a variation of ± 5%, ± 10%, ± 20%, or ± 25% of the value specified. For example, "about 50" percent may in some embodiments carry a variation from 45 to 55 percent. For integer ranges, the term "about" may include one or two integers greater than and/or less than a recited integer at each end of the range. Unless indicated otherwise herein, the term "about" is intended to include values, e.g., weight percents, proximate to the recited range that are equivalent in terms of the functionality of the individual ingredient, the composition, or the embodiment.

As will be understood by the skilled artisan, all numbers, including those expressing quantities of ingredients, properties such as molecular weight, reaction conditions, and so forth, are approximations and are understood as being optionally modified in all instances by the term "about." These values may vary depending upon the desired properties sought to be obtained by those skilled in the art utilizing the teachings of the descriptions herein. It is also understood that such values inherently contain variability necessarily resulting from the standard deviations found in their respective testing measurements.

As will be understood by one skilled in the art, for any and all purposes, particularly in terms of providing a written description, all ranges recited herein also encompass any and all possible sub-ranges and combinations of sub-ranges thereof, as well as the individual values making up the range, particularly integer values. A recited range (e.g., weight percents or carbon groups) includes each specific value, integer, decimal, or identity within the range. Any listed range may be easily recognized as sufficiently describing and enabling the same range being broken down into at least equal halves, thirds, quarters, fifths, or tenths. As a non-limiting example, each range discussed herein may be readily broken down into a lower third, middle third and upper third, etc. As will also be understood by one skilled in the art, all language such as "up to", "at least", "greater than", "less than", "more than", "or more", and the like, include the number recited and such terms refer to ranges that may be subsequently broken down into sub-ranges as discussed above. In the same manner, all ratios recited herein also include all sub-ratios falling within the broader ratio. Accordingly, specific values recited for radicals, substituents, and ranges, are for illustration only; they do not exclude other defined values or other values within defined ranges for radicals and substituents.

One skilled in the art will also readily recognize that where members are grouped together in a common manner, such as in a Markush group, the invention encompasses not only the entire group listed as a whole, but each member of the group individually and all possible subgroups of the main group. Additionally, for all purposes, the invention encompasses not only the main group, but also the main group absent one or more of the group members. The invention therefore envisages the explicit exclusion of any one or more of members of a recited group.

Accordingly, provisos may apply to any of the disclosed categories or embodiments whereby any one or more of the recited elements, species, or embodiments, may be excluded from such categories or embodiments, for example, as used in an explicit negative limitation.

The term "contacting" refers to the act of touching, making contact, or of bringing to immediate or close proximity, including at the cellular or molecular level, for example, to bring about a physiological reaction, a chemical reaction, or a physical change, e.g., in a solution, in a reaction mixture, in vitro, or in vivo.

An "effective amount" refers to an amount effective to treat a disease, disorder, and/or condition, or to bring about a recited effect. For example, an amount effective may be an amount effective to reduce the progression or severity of the condition or symptoms being treated.

Determination of a therapeutically effective amount is well within the capacity of persons skilled in the art. The term "effective amount" is intended to include an amount of a compound described herein, or an amount of a combination of compounds described herein, e.g., that is effective to treat or prevent a disease or disorder, or to treat the symptoms of the disease or disorder, in a host. Thus, an "effective amount" generally means an amount that provides the desired effect.

The terms "treating", "treat" and "treatment" include (i) preventing a disease, pathologic or medical condition from occurring (e.g., prophylaxis); (ii) inhibiting the disease, pathologic or medical condition or arresting its development; (iii) relieving the disease, pathologic or medical condition; and/or (iv) diminishing symptoms associated with the disease, pathologic or medical condition. Thus, the terms "treat", "treatment", and "treating" extend to prophylaxis and include prevent, prevention, preventing, lowering, stopping or reversing the progression or severity of the condition or symptoms being treated. As such, the term "treatment" includes both medical, therapeutic, and/or prophylactic administration, as appropriate.

The terms "inhibit", "inhibiting", and "inhibition" refer to the slowing, halting, or reversing the growth or progression of a disease, infection, condition, or group of cells. The inhibition may be greater than about 20%, 40%, 60%, 80%, 90%, 95%, or 99%, for example, compared to the growth or progression that occurs in the absence of the treatment or contacting.

The term "inulin" is well known in the art and refers to α-D-glucopyranosyl-[α-D-fructofuranosyl](n-1)-D-fructofuranoside, a polysaccharide consisting of a family of linear β-D (2->1) polyfructofuranosyl α-D-glucoses, in which an unbranched chain of typically up to about 100 fructose moieties (n = about 5 to about 100 for plant-derived material and about 5 to about 100,000 for microbe-derived material) is linked to a single terminal glucose unit.

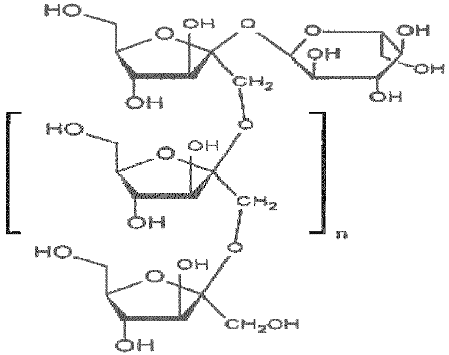

Inulin is a plant-derived polysaccharide and has a relatively hydrophobic, polyoxyethylene-like backbone. This structure and its non-ionized nature allow recrystallization and preparation in a very pure state. Inulin may be prepared as molecularly polydisperse at molecular weights up to about 16 kDa. Suitable inulin particles (such as the β-In and InAc particles described herein) may be prepared from raw inulin having a molecular weight of about 2 kDa to about 12 kDa, about 3 kDa to about 8 kDa, about 4 kDa to about 6 kDa, or about 5 kDa.

Inulin acts as the storage carbohydrate of Compositae (or Asteraceae) and is obtained in high molecular weights from dahlia tubers. Inulin may be obtained commercially from a variety of suppliers such as Sigma (St. Louis, MO). Inulin exists in several distinct forms (polymorphs), including the alpha, beta, gamma, delta, and epsilon forms (see, e.g., WO 2011/032229 (Petrovsky et al.)). These forms may be differentiated by their solubility parameters. Alpha inulin (α-In) and beta inulin (β-In) may be prepared by precipitation from water and ethanol, respectively. Both alpha and beta isoforms are substantially soluble in water at 37°C. Gamma inulin (γ-In) is insoluble in water at 37 °C but is soluble in water at high concentrations (>50 mg/mL) at 70-80 °C. Inulin in a β-polymorphic form or in any water soluble form has never been shown to have an adjuvant/immune-potentiating effect.

The term "inulin acetate" (InAc) refers to acetylated inulin. Typically at least about 90% of available hydroxyl groups of the inulin are acetylated, and often at least about 95% or at least about 98%. In embodiments, inulin acetylated to a lesser degree may be used, such as inulin with at least about 10% of available hydroxyl groups acetylated. In embodiments, at least about 25%, at least about 50%, or at least about 75% of the available hydroxyl groups may be acetylated for various inulin acetate formulations. In embodiments, acetylated inulin is considered to be inulin acetate when the hydroxyl peak of its infra-red spectrum disappears and acetyl peaks appear. Inulin acetate is insoluble in water even at elevated temperatures, but is soluble in various organic solvents such as acetone, ethyl acetate, chloroform, dichloromethane, and the like.

Inulin acetate does not activate the alternate complement pathway (APC), and InAc does not function as vaccine adjuvant when co-injected with an antigen. InAc is readily dispersible in saline. An antigen must be encapsulated inside InAc particles or physically associated with InAc particles to function as a vaccine adjuvant. InAc microparticles and nanoparticles may function as vaccine adjuvants by enhancing the uptake of the antigen and delivering the encapsulated antigen to relevant compartments of immune cells for activation. InAc is also a novel TLR agonist, as shown for the first time herein. While not being bound by theory, this suggests that InAc works as a vaccine adjuvant by a different mechanism than non-acetylated inulin forms.

Other inulin derivatives may be used in addition to, or in place of, inulin acetate. An inulin derivative may be inulin where hydroxyl groups of the inulin are modified by chemical substitution with alkyl, aryl, or acyl groups, or by oxidation or reduction, by known methods. See, for example, techniques described by Greg T. Hermanson in Bioconjugate Techniques, Academic Press, San Diego, CA (1996).

Inulin derivatives include ester linkages, ether linkages, amide linkages, carbamate linkages, oxidized or reduced forms of inulin and their derivatives, cationic/anionic/non-ionic modifications of inulin (anionic: O-(carboxymethyl)inulin; cationic: Inutec H25P), and complexation of inulin or its oxidized/reduced form with other agents (e.g., complexation of oxidized inulin with heavy metals such as copper, zinc, cadmium, or the like).

An "inulin ester" or "esterified inulin" refers to inulin esterified on hydroxyl groups by condensation with ester-forming groups such as carboxylic acids or acylation with groups such as carboxylic anhydrides, or the like. Examples of inulin ester include but are not limited to inulin propanoylate, inulin butanoylate, inulin phosphates, and the like.

An "inulin ether" or "etherified inulin" refers to inulin etherified on hydroxyl groups with ether-forming groups such as groups having appropriate leaving groups such as halides, acid halides, or the like. Examples of inulin ethers include but are not limited to methylated inulin (e.g., inulin per-methyl ether), ethylated inulin, and the like.

Examples of oxidized or reduced forms of inulin and their derivatives include inulin carbonate, dialdehyde inulin, and the like. Other inulin derivatives that may be used include cyanoethyl inulin, amino-3-oxopropyl-inulin, carboxyethyl inulin, hydroxyimino-3-aminopropyl inulin, inulin carbamates (e.g., Inutec SP1), or a combination thereof.

The inulin derivatives may be in the form of particulate formulations where the cargo molecules (e.g., an antigen) are encapsulated within the particles, or coated or conjugated on to the particles. The modification of the available hydroxyl groups of the inulin may be to a degree as described above for the acetylation of inulin acetate.

"Active agent" or "active" refers to a drug, immunological agent (e.g., an antigen), or other cargo molecule that may be encapsulated or physically associated with beta-inulin or InAc particles. "Physically associated" refers to an association by electrostatic interactions, including hydrophilic, hydrophobic interactions, or hydrogen bonding to a polymer or to particles. "Physically associated with a particle" may refer to the particle being coated with the agent, as well as the particle being covalently conjugated to the agent.

Methods of conjugation are well known in the art and several techniques are described by Greg T. Hermanson in Bioconjugate Techniques, Academic Press, San Diego, CA (1996). The active agent may be loaded into particles at about 1 wt.% to about 25 wt.%, about 1 wt.% to about 10 wt.%, or about 1 wt.% to about 5 wt.% of the combined particles and cargo and/or physically associated molecules.

"Antigen" refers to any substance which is capable, under appropriate conditions, of inducing a specific immune response and of reacting with the products of that response; that is, with specific antibody or specifically sensitized T lymphocytes, or both. The terms "antigen," "immunogenic agent," and "active agent" may be used interchangeably throughout this document. Antigens may be soluble substances, such as toxins and self or foreign proteins/peptides, or particulate, such as bacteria, viruses, and tissue cells; however, only a small portion of the protein or polysaccharide molecule known as the antigenic determinant or epitope is recognized by the specific receptor on a lymphocyte. Similarly the antibody or effector lymphocyte produced by the response combines only with the one antigenic determinant. A bacterial cell or large protein may have many hundreds of antigenic determinants, some of which are more important than others in protective immunity.

A partial list of known antigens that may be used with the adjuvants provided herein is as follows: allogenic antigens, which occur in some but not all individuals of the same species, e.g., histocompatibility antigens and blood group antigens, formerly called isoantigen; bacterial antigens; blood group antigens, which are present on the surface of erythrocytes and vary between individuals of the same species and are used as the basis for blood typing; capsular antigens; K, L and V antigens; carcinoembryonic antigen (CEA); oncofetal antigen; common antigens, which are antigenic determinants present in two or more different antigen molecules and the basis for cross-reactions among them; complete antigens, which are antigens that both stimulate the immune response and react with the products, e.g. antibody, of that response; conjugated antigens; haptens; cross-reacting antigens are antigens that combine with antibody produced in response to a different but related antigen, owing to similarity of antigenic determinants, or identical antigens in two bacterial strains, so that antibody produced against one strain will react with the other; dog erythrocyte antigens (DEA), which are antigens found on dog erythrocytes and used to distinguish different blood groups in the species; environmental antigens, those found in pollens, fungi, house dust, foods and animal dander; feline oncomavirus cell membrane antigen (FOCMA), which is a tumor-specific antigen present on the membrane of cells in cats infected with feline leukemia virus; flagellar antigens; H or Hauch antigens, which are antigens that occur in bacterial flagella; flea antigens, which comprise some components of flea saliva, as well extracts of fleas; Forssman antigens; heterophil antigens, which are those antigens occurring in various unrelated species, mainly in the organs but not in the erythrocytes or only in the erythrocytes or occasionally in both the organ and the erythrocytes; group specific (gs) antigens, which are common to a certain group of organisms, e.g. streptococci, oncornaviruses; heterogeneic antigens; xenogeneic antigens; heterophil antigen; heterogenetic antigen, which includes an antigen capable of stimulating the production of antibodies that react with tissues from other animals or plants; hidden antigens, which are antigens that are not normally exposed to circulating lymphocytes, e.g., within central nervous tissue, testicular tissue and certain intracellular components, and thus they do not normally evoke an immune response; histocompatibility antigens; H-Y antigens, which are histocompatibility antigens of the cell membrane; Ia antigens, which are histocompatibility antigens governed by the I region of the major histocompatibility complex (MHC), located on B lymphocytes, T lymphocytes, skin, and certain macrophages; isogenic antigens, which are antigens carried by an individual, or members of the same inbred strain, capable of eliciting an immune response in genetically different individuals of the same species, but not in individuals bearing it; K antigens; bacterial capsular antigens; L antigens, which are capsular antigens of Escherichia coli; Ly antigens, which are antigenic cell-surface markers of subpopulations of T lymphocytes, classified as Ly 1, 2 and 3; lymphocyte-defined (LD) antigens; class II antigens found in lymphocytes, macrophages, epidermal cells and sperm; M antigens, type-specific antigens that appear to be located primarily in the cell wall and are associated with virulence of Streptococcus pyogenes; Marek's tumor specific antigen (MATSA), found on the surface of cells infected by Marek's disease, herpesvirus; Negre antigen, which is an antigen prepared from dead, dried and triturated tubercle bacilli by means of acetone and methyl alcohol; nuclear antigens, which are the components of cell nuclei with which antinuclear antibodies react; 0 antigen, which occurs in the cell wall of bacteria; oncofetal antigen, which is a gene product that is expressed during fetal development, but repressed in specialized tissues of the adult and is also produced by certain cancer, includes alpha-fetoprotein and carcinoembryonic antigen; organ-specific antigen, which is any antigen that occurs exclusively in a particular organ and serves to distinguish it from other organs; partial antigens; pollen antigen, the essential polypeptides of the pollen of plants; private antigens, which are antigens of the low-frequency blood groups; recall antigens, which are antigens to which an individual has previously been sensitized and which is subsequently administered as a challenging dose to elicit a hypersensitivity reaction; sequestered antigens, which are certain antigens that are sequestered anatomically from the immune system during embryonic development and thus believed not to be recognized as "self' and should such antigens be exposed to the immune system during adult life, an autoimmune response would be elicited; serologically defined (SD) antigen, which is a class I antigen of the major histocompatibility complex, identifiable by the use of specific antisera; synthetic antigen, a chemically synthesized or produced by recombinant DNA technology, the synthesis of polymers, based on sequences found in microbial or other antigens; T-dependent antigen (the immune response of most antigens requires T helper (Th) lymphocytes; lymphokines produced by T lymphocytes determine the characteristics of antibodies produced, which may change during the immune response); thymus-dependent antigen, an antigen that requires T lymphocyte participation before an immune response can occur; thymus-independent antigen, an antigen that elicits an antibody response without the participation of T lymphocytes; tolerogenic antigen; tumor-specific antigen (TSA), which are antigens found only in tumor cells; V antigen, Vi antigen, which are antigens contained in the capsule of a bacterium and thought to contribute to its virulence; xenogeneic antigen, which are common to members of one species but not to members of other species; called also heterogeneic antigen.

The "complement pathway" refers to a complicated enzyme cascade made up of numerous serum glycoproteins that normally exist in in-active, proenzyme form. The system has three distinct pathways: "the classical pathway," "the alternative pathway," and "lecithin pathway." The classical pathway of the complement system is a major effector of the humoral branch of the human immune response. The trigger for the classical pathway is either IgG or IgM antibody bound to antigen. Binding of antibody to antigen exposes a site on the antibody which is a binding site for the first complement component, Cl.

The "alternative complement pathway" or APC does not require antibody for its activation. Rather, a variety of antigens such as bacterial lipopolysaccharide (LPS) and components of viruses and other pathogens have the ability to activate this pathway. While not being bound by theory, it is thought to have evolved earlier than the classical complement pathway, which depends on the relatively recently evolved antibody molecule. Like the classical pathway, the alternative pathway produces both a C3 and a C5 convertase, which leads to the production of C5b and then to the formation of the membrane attack complex (MAC). However, the specific molecular players and the path followed along the way are, however, different than those of the classical complement pathway.

While the stimulating factors for each pathway are distinct, each one has a similar terminal sequence which creates the membrane attack complex (MAC), an enzyme complex which perforates various cell surfaces. In addition, both the alternative and classical pathways have as their by-products a number of anaphylatoxins - small peptides which contribute to an inflammatory response.

Molecules involved in the complement system are generally given the name "C" and then a number, for example "Cl". The numbers are not indicative of the order in which they act within the cascade, but refer to the order in which they were discovered. Complement proteins normally exist in proenzyme form, and are activated sequentially by successive cleavages of the various molecules. When a complement protein is split, two fragments are formed, generally referred to as "a" and "b." Complement molecule C5, for example, is cleaved into fragments C5a and C5b.

Ovalbumin, or "ova", is a water-soluble albumin and is the primary component of chicken egg white. Approximately 60-65% of the total protein in an egg white is ovalbumin. Ovalbumin may act as a storage protein and the ovalbumin of chicken eggs may be used as an antigen to stimulate allergic reactions in test subjects.

The term "animal", as used herein, refers to any of a kingdom of living things including, but not limited to a member of Kingdom Animalia, including many-celled organisms, and the single-celled organisms that typically differ from plants in 1) having cells without cellulose walls, 2) lacking chlorophyll and the capacity for photosynthesis, 3) requiring more complex food materials, for example, proteins, 4) being organized to a greater degree of complexity, and 5) having the capacity for spontaneous movement and rapid motor responses to stimulation. The term animal, as used herein, also refers to any of Kingdom Animalia grand divisions, or subkingdoms, and the principal classes under them, including, but not limited to: Vertebrata, including Mammalia or mammals, including Ayes or birds, Reptilia, Amphibia, Pisces or fishes, Marsipobranchiata (Craniota); and Leptocardia (Acrania); Tunicata, including the Thaliacea, and Ascidioidea or ascidians; Articulata or Annulosa, including Insecta, Myriapoda, Malacapoda, Arachnida, Pycnogonida, Merostomata, Crustacea (Arthropoda); and Annelida, Gehyrea (Anarthropoda); Helminthes or vermes, including Rotifera, Chaetognatha, Nematoidea, Acanthocephala, Nemertina, Turbellaria, Trematoda, Cestoidea, Mesozea. Molluscoidea, including Brachiopoda and Bryozoa; Mollusca, including Cephalopoda, Gastropoda, Pteropoda, Scaphopoda, Lamellibranchiata or Acephala; Echinodermata, including Holothurioidea, Echinoidea, Asterioidea, Ophiuroidea, and Crinoidea; Coelenterata, including Anthozoa or polyps; Ctenophora and Hydrozoa or Acalephs; Spongiozoa or Porifera, including the sponges; and Protozoa, including Infusoria and Rhizopoda. The term animal, as used herein, further refers to the following non-limiting examples: human, primate, dog, cat, cow, lamb, pig, hog, poultry, horse, mare, mule, jack, jenny, colt, calf, yearling, bull, ox, sheep, goat, llama, bison, buffalo, lamb, kid, shoat, hen, chicken, turkey, duck, goose, ostrich, other birds or fowl, rabbit, hare, guinea pig, hamster mouse, rat, other rodents, fish and other aquatic species, and amphibians. The term "animal" as used herein additionally refers to transgenic animals.

The term "subject" as used herein means an animal that is the object of medical or scientific study.

The Bicinchoninic Acid (BCA) Protein Assay is a biochemical assay for determining the total level of protein in a solution (e.g., 0.5 µg/mL to 1.5 mg/mL). The total protein concentration is exhibited by a color change of a sample solution from green to purple in proportion to protein concentration, which can then be measured using colorimetric analysis techniques.

Since 1926, alum has been the only approved adjuvant for human use in the U.S. but it produces only humoral immune responses (Th2 type). In addition, alum-adjuvant vaccines have many disadvantages, including the loss of potency upon conventional lyophilization and freezing (Maa et al., J. Pharm. Sci. 2003; 92(2):319-32). Cold storage of a number of currently available vaccines is a serious concern for pharmaceutical companies and providers, especially in developing countries.

Provided in the embodiments described herein are efficient adjuvant formulations capable of stimulating both arms of the immune system including humoral (for extracellular pathogens) and cellular (for intracellular pathogen). Additionally, the disclosure provides vaccine and delivery formulations that may be physically stable at ambient temperatures and suitable for freeze-drying, therefore eliminating the requirement for cold-chain storage. Further, inulin acetate is a novel TLR agonist and stimulates the immune system when formulated as a particulate. Some of the many other advantages of using InAc as a vaccine adjuvant are provided herein.

The disclosure provided below details the preparation of nanoparticle and microparticle formulations of the water-soluble β-polymorphic form of inulin (β-In) and its synthetic derivative inulin acetate (InAc) and the use of these formulations as immunopotentiators. Ovalbumin (ova) may be used as a model antigen. β-In or InAc microparticles containing ova may be prepared by a double emulsion-solvent evaporation method. In-vitro release studies show that most of the incorporated ova (>95 %) is released from β-In particles and InAc particles within 16 hours and 528 hours (22 days), respectively.

Immunization studies in mice show that ova encapsulated in β-In microparticles produced stronger antibody responses (only Th2 type) than unencapsulated (free) ova. This result was similar in type of response and greater in intensity compared to the group where alum (the only FDA approved adjuvant) is used as an adjuvant. For example, the intensity of the response may be up to about 4 times, or about 2 to about 4 times the intensity of the response of the group where alum is used as an adjuvant. However, ova containing InAc microparticles generated significantly higher antibody responses than alum bound ova.

For example, the total IgG response in mice receiving ova-containing InAc microparticles was up to about 90 times greater than in mice receiving alum bound ova. Additionally, the IgG1 response in mice receiving ova-containing InAc microparticles was up to about 75 times greater than in mice receiving alum bound ova. Further, the IgG2a response in mice receiving ova-containing InAc microparticles was up to about 1200 times greater than in mice receiving alum bound ova. Accordingly, ova loaded InAc particles may generate antibody responses that are significantly higher than other adjuvants bound to ova. For example, the ova loaded InAc particles can generate significantly higher titers than alum bound ova as follows: Total IgG: at least about 5-100 times higher, at least about 10-150 times higher, or at least about 10-200 times higher; IgG1: at least about 10-100 times higher, at least about 15-85 times higher, or at least about 20-80 times higher; and IgG2a: at least about 2-1500 times higher, at least about 100-1500 times higher, or at least about 500-1500 times higher.

Significantly, InAc microparticles generated both Th1 and Th2 type immune responses, which are desired in modern vaccine delivery technology. The fact that examples as disclosed were carried out by an intradermal (i.d.) route further emphasizes the potential use of InAc particles as adjuvants in vaccine delivery technology such as micro-needles, or other delivery methods that use the i.d. route.

The present disclosure also demonstrates the extent of immune response that may be manipulated by modulating the dose, particle size (nano vs. micro) and route of administration. Different sizes of InAc particles may be prepared by the use of a select combination of buffers, surfactants, solvents and other excipients. These multiple formulations have the potential for broad uses due to their immunostimulatory effects. The compositions described herein may be used to provide a novel and cost-effective vaccine adjuvant with significantly high (cellular and humoral) immune response properties and improved safety profiles.

The adjuvant formulations described herein provide stronger immune responses than alum as an adjuvant. The adjuvant formulations of the invention stimulate the production of both humoral mediated immunity (Th2; for extracellular pathogens) and cellular mediated immunity (Thl; for intracellular pathogens), which is a significant benefit over other adjuvants, including alum. Additionally, current vaccines may lose potency upon conventional lyophilization and freezing. The vaccine and delivery formulations as disclosed may be physically stable at ambient temperatures and suitable for freeze-drying, therefore eliminating the requirement for cold-chain storage or the addition of preservatives.

The adjuvant formulations as provided herein are biocompatible and biodegradable. Inulin has a long history of safe use in humans with the advantage of having a non-toxic profile. The metabolites of inulin are fructose and glucose, which can be easily excreted. Inulin has been reported to be non-toxic and has been used as a food additive.

The adjuvant formulations described herein are derived from plants. Due to their plant origin, these adjuvant formulations, including InAc, may be universally used. This is in sharp contrast to substances derived from animals, which are not used or consumed by vegetarians. Furthermore, because they are derived from plants, the adjuvant formulations provided herein do not carry a risk of microbial or blood contamination from animals.

The adjuvant formulations described herein provide enhanced stability over currently available options. Commercial vaccines require cold storage, which results in high costs and increased logistics associated with cold storage and transport of vaccines. The use of InAc microparticles or nanoparticles as adjuvants provides vaccine formulations that are physically stable at ambient temperatures as well as physically stable after freeze-drying.

This increased stability eliminates the costly and complex requirement for cold-chain monitoring and storage. Certain adjuvants, such as gamma-inulin, require co-injection of an antigen solution for immunoadjuvant effect. The co-injection of antigen and adjuvant can make the antigen susceptible to aggregation or degradation. Antigens that have characteristics that make them unstable to light, oxygen or moisture can also be protected by encapsulation in the InAc microparticle or nanoparticle adjuvants to improve shelf-life.

The adjuvant formulations described herein are cost effective to produce. InAc can be prepared from any form of inulin including commercially available raw inulin. The adjuvant formulations described herein may be prepared at a significantly lower cost than other sophisticated vaccine delivery systems. By preparing InAc as provided herein, complex procedures to prepare different forms of inulin may be avoided and polymorphic conversions between different isoforms are not a concern. Remarkably, the yield of InAc preparation is near 100%, which is highly favorable as compared to yields of 10-50% for preparing gamma-inulin.

The adjuvant formulations described herein provide superior dispersibility and dynamics, as well as a uniform particle size. Dispersibility is an important property of a vaccine formulation with respect to its preparation, handling, and administration from multi-dose vials. The InAc microparticles or nanoparticles were optimized for easy re-dispersibility by using adequate surfactants and cryoprotectants. Therefore, the InAc formulations provided herein may be uniformly dispersed in an aqueous solvent or buffer. The InAc microparticles or nanoparticles may be uniformly dispersed in aqueous solution upon shaking and therefore, they may be readily administered using standard procedures from multi-dose vials.

The adjuvant formulations described herein provide for extended release of antigen. Antigen may be released from the InAc formulations described herein for extended periods of time (including, but not limited to 1-2 months, 2-3 months, or greater). Because of the extended time period for antigen release, the formulations of the invention may be used as single shot vaccines without the need for administration of booster doses. By eliminating the need for multiple doses of a vaccine, the formulations described herein provide significant reductions in cost and are more convenient for the patient and provider. There are other advantages created by the elimination of the need for greater than one vaccine injection. For example, a vaccination campaign may be more successful when patient compliance is increased. Further, because the size of the particles of the formulations described herein may be manipulated (nano vs. micro), the desired delivery destination in the subject may also be selected, which in turn allows for greater precision in the resulting immune response.

The disclosure provides beta-inulin and inulin acetate as novel vaccine adjuvant and delivery formulations that are non-toxic and biocompatible. Inulin has a long history of safe use in humans and its metabolic products (fructose and glucose) are readily excreted. Preliminary observations indicate no visible or histological toxicities associated with InAc and InAc formulations at the site of injection in mice. If any local inflammation or irritation at the site of injection appears in humans, reducing the amount of adjuvant incorporated will substantially avoid this problem. To accommodate a required antigen dose in reduced amounts of beta-inulin or InAc particles, the antigen loading may increased by manipulating the formulation and process parameters while preparing the nano/micro particles.

Several methods may be used including solvent/nonsolvent, single emulsion and double emulsion preparatory techniques. Micro/nano particles prepared by double emulsion method were found to have an advantageous combination of high antigen loading, low burst release (percentage of antigen released from particles in the first 30 minutes), and increased sustained release, under similar formulation conditions tested. However, by changing the formulation parameters, the loading of the antigen could be further enhanced beyond the loading achieved by any known method. The loading of antigen into InAc micro/nano particles may be further increased by increasing the antigen:polymer ratio in the formulation (0.4 µg/mg particles to 51 µg/mg particles by increasing the loading conditions from about 100 µg antigen per about 100 mg InAc polymer to about 20 mg antigen per about 100 mg InAc polymer. This ratio may be increased to about 75 µg/mg of InAc particles or about 100 µg/mg of InAc particles, with optimized formulating factors. The loading of an antigen inside nano/microparticles can be further enhanced by incorporating mannitol with the antigen in the loading procedure. Antigen amounts of up to about 100 µg/mg of InAc particles can be loaded in the presence of mannitol. Similarly, other carbohydrate or hydrophilic substances (e.g., trehalose) may be added to the formulation to enhance the loading of an antigen inside InAc micro/nano particles.

The antigen loading efficiency into β-inulin is significantly higher. Loading of up to approximately 500 µg of antigen per mg (e.g., 100 µg, 200 µg, 300 µg, 400 µg, or 500 µg of antigen/mg (β-inulin) may be achieved. Thus, the formulations provided herein impart the ability to tailor the delivery of the antigen as needed to meet the objectives of the treatment, the particularities of the antigen, and/or the unique needs of the subject.

The following are provided as non-limiting examples of some of the research and commercial applications of the compositions and formulations of the invention.

Inulin and InAc particles function as both vaccine delivery systems and vaccine adjuvants. Strong and safe vaccine adjuvants are scarce. As a non-limiting example, cancer vaccines and vaccines against extracellular pathogens, and intracellular pathogens (such as viruses and parasites) are just a few of the examples of vaccines where the use of the formulations of the invention would be beneficial in order to active both the Th1 and Th2 types of immune response. The technology provided in this invention provides a superior and safer approach than the existing technology (alum) in stimulating both arms of immune response. The compositions and formulations of this invention can be utilized as human or animal vaccine adjuvants for a diverse and very broad range of diseases and conditions.

The adjuvant formulations described herein may be combined with other immunostimulatory and/or immunomodulatory agents such as cytokines, or other regulatory proteins, including but not limited to lymphokines and interleukins, or products of the immune system cells or other cells, as well as those products of the immune system cells or other cells that act as intercellular mediators in the modulation of responses such as immune responses or the products of pathogens that non-specifically stimulate the immune system such as pathogen associated molecular patterns (PAMS) or any ligands for pattern recognition receptors (PRRs). Suitable examples include, but are not limited to, CpG, or other TLR agonists, interleukins IL-1 through IL-35 and others, interferons (all types, including type 1 and type 2), which may be used with the adjuvant formulations described herein to further modulate and enhance the immune response.

The adjuvant formulations described herein may be combined with other therapies for drug or pharmaceutical delivery in a subject. For example, an adjuvant formulation may be administered in combination with chemotherapeutic agents (e.g., bleomycin, doxorubicin, paclitaxel, 5-fluorouracil, vincristine, and the like) for cancer treatment, drugs such as donepezil, galantamine, memantine, rivastigmine, or tacrine for Alzheimer's therapy, or with photodynamic therapy and/or dietary supplements (e.g., curcumin, omega-3 fatty acids, vitamin C, and the like) for other therapies.

The adjuvant formulations described herein may be used for vaccine purposes and provide for the delivery of a variety of antigens. The compositions and formulations described herein may be used to deliver antigens including, but not limited to, viral antigens, subunit vaccines, tumor antigens, allergies as antigens, nucleic acid vaccines, including but not limited to DNA or RNA vaccines, recombinant proteins and recombinant antigens, as well as protein/carbohydrate/polysaccharide antigens.

The adjuvant formulations described herein can be used with antigens, pharmaceuticals or proteins, or any other treatment compound or formulation, for the treatment and prevention of a wide variety of ailments and conditions. The novel compositions and formulations described herein may be used as a therapy, prophylaxis, or vaccines against conditions affecting humans, mammals and other animals, and other living organisms.

The compositions and formulations of the invention may be used in combination with antigens, other agents, pharmaceuticals, proteins, or any other suitable compound or formulation as a therapy, prophylaxis, or vaccine for diseases, including but not limited to malaria, influenza A and B, other influenza and variants thereof such as seasonal influenza, Swine influenza, para-influenza, pandemic influenza, resistant pandemic influenza, Avian influenza, hepatitis A, hepatitis B, hepatitis C, Anthrax, Diphtheria, Haemophilus influenzae type b (Hib), AIDS, Encephalitis, Japanese Encephalitis (JE), Lyme Disease, Malaria, Marburg virus, Measles, Monkeypox, Mumps, Pertussis (Whooping Cough), Rubella (German Measles), Poliomyelitis (Polio), Rabies, Rotavirus, Smallpox, Tetanus (Lockjaw), Tuberculosis, Typhoid, Yellow Fever, tropical diseases, Parasitic diseases, Leishmaniasis, Conformational Disorders, Sarcocystis, Chronic Fatigue Syndrome, Haemorrhagic fevers, Leptospirosis, Botulism, Dengue Fever, Q fever, Babesiosis, Legionella, Trypansomiasis, Leprosy, Lyme disease, and Rocky mountain spotted fever.

The compositions and formulations of the invention may be used in combination with antigens, other agents, pharmaceuticals, proteins, or any other suitable compound or formulation as a therapy or prophylaxis for, or vaccine for diseases, including but not limited to those diseases or conditions caused by Giardia species, Streptococcal species, Staphylococcus species, Escherichia species, Enterobacteriaceae species, Enterococcal species, Haemophilus species, Mycobacterium species, Myxobactierum species, Neisseria species, Plasmodium species, Pseudomonas species, Salmonella species, Shigella species, Meningococcal species, Leptospira species, Candida species, Copodella species, Yeast species, Fungal species, Cryptococcus species, Bartonella species, Rickettsia species, Borrelia species, Trypanosomiasis species, Campylobacter species, Rotavirus, HIV, AIDS, Avian Influenza Virus, Herpes virus, including Shingles (Herpes zoster) and HSV (Herpes simplex virus), Human Papillomavirus (HPV), Hendra virus, human metapneumoviruses, rhinoviruses, bocaviruses, coronaviruses, Invasive saffold virus, Respiratory synsitial virus (RSV), Hantavirus, Hemorrhagic Fever virus, Vaccinia virus, SARS, West Nile Virus; Zoonotic diseases, including, but not limited to Bovine Spongiform Encephalitis (BSE), and Nipah virus, Brucellosis, rabies and parasitic diseases, including but not limited to cysticercosis/taeniasis and echinococcosis/hydatidosis, animal influenzas, neglected zoonotic diseases, Ruminant diseases, PRRS, Porcine epidemic diarrhea, Ehrlichiosis, Bluetongue, Chronic wasting disease, Classical swine fever, Contagious equine metritis, Equine herpesvirus, Equine infectious anemia, Equine piroplasmosis, Equine viral arteritis, Foot-and-mouth disease, Johnes disease, Piroplasmosis, Pseudorabies, Scrapie, Spring viremia carp, Vesicular stomatitis; Foodborne diseases, diseases caused by Prions, Creutzfeldt Jakob disease (CJD) and variant Creutzfeldt-Jakob disease (vJD), Coxsackievirus species; Tick borne diseases, Mosquito-borne diseases, Bat-borne diseases, Rodent-borne diseases, Avian-borne diseases, and diseases resistant to antifungal agents and antimicrobial agents.

Additionally, the adjuvant formulations described herein may be used in combination with antigens, other agents, pharmaceuticals, proteins, or any other suitable compound or formulation for the prevention or treatment of cancer, or for the mitigation of cancer symptoms and side effects, such as in the treatment of conditions such as autoimmune disorders and diseases, diseases affecting memory, including but not limited to dementia and Alzheimer's disease, diseases affecting motor function, Crohn's disease, diseases of the gastrointestinal tract, and genetic disorders and diseases. The formulations and compositions described herein may be used for delivery of tumor associated antigens for treatment of cancer and or other malignancies or mitigation of symptoms and side-effects associated with the same. Recombinant DNA antigens may be successfully delivered with the formulations and compositions of the invention as a potent vaccine formulation.

The compositions, formulations and methods of the invention may be used in the production of an immune response or immune responses in one or more humans or animals. The compositions and methods can be used in the production of quantities of antibodies from animals, or from the products of animals (e.g., eggs), or components of animals (for example, blood, lymphatic fluid, tissue, cells and other components derived from animals), for use in research, commercial products, assays, medicine and medical treatments, vaccines, and other areas of interest. The compositions and methods may also be used in the production of, for example, monoclonal antibodies, polyclonal antibodies, and antisera, as well as any other desired products of immune systems or immune cells.

The adjuvant formulations of the invention allow different routes of administration: The compositions and formulations of the invention may be administered by a variety of different routes of administrations including parenteral (e.g., subcutaneous (SC), intramuscular (IM), or intravenous (IV)), transdermal (TD), intrarectal (IR), intranasal (IN), pulmonary, intraocular (IO), intragastric (IG), intravaginal (1VG), intratratracheal (IT), sublingual, buccal, and/ or oral.

### Beta-Inulin and Inulin Acetate Microparticles and Nanoparticles

The compositions described herein may be formulated in a pharmaceutically acceptable carrier, diluent or excipient in a form suitable for injection, or a form suitable for oral, rectal, vaginal, topical, nasal, pulmonary, or ocular administration (e.g., a pharmaceutical composition). The compositions may include one or more an active agents such as, for example, a vaccinating antigen (including recombinant antigens), an antigenic peptide sequence, or an immunoglobulin.

In embodiments, a method of stimulating an immune response in a subject, for the purposes of, for example, preventing, treating, or inhibiting an infectious disease, autoimmune disease, immunodeficiency disorder, neoplastic disease, degenerative disease, or aging disease is disclosed, where the method includes administering to a subject an effective amount of a formulation described herein.

In embodiments, a method of enhancing an immune response in a subject, for the purposes of, for example, preventing, treating, or inhibiting an infectious disease, autoimmune disease, immunodeficiency disorder, neoplastic disease, or degenerative disease, or aging disease is disclosed, where the method includes administering to a subject an effective amount of a formulation described herein.

The term "substantially purified" as used herein is to be understood as referring to an inulin preparation that is essentially free of other polysaccharides and/or other exogenous biological materials (e.g., microbial- or plant-derived materials). The formulations described herein are typically purified or substantially purified. Such formulations will comprise no more than about 10% (by weight) or no more than about 5% (by weight) of exogenous biological materials, and may be prepared by any of the methods well known to persons skilled in the art including well known hot water extraction and purification processes employed in the commercial production of inulin from chicory (Stephen, A.M. et al. (Eds.), Food Polysaccharides and their Applications, 2nd Ed., CRC Press, Boca Raton, FL (2006)).

The compositions may include various active agents such as vaccinating antigens, antigenic peptide sequences, immunoglobulins, or combinations thereof. Alternatively, or additionally, the active agent may be a lymphokine or cytokine, a thymocyte stimulator, a macrophage stimulator, an endotoxin, a polynucleotide molecule (e.g., encoding a vaccinating agent), CpGs, or recombinant viral vector, a whole microorganism (e.g., a bacterial lysate), a whole virus (e.g., an inactivated or attenuated virus), or a combination thereof. The compositions described herein may be used with inactivated/attenuated whole virus as the active component. Additional examples of agents that may be combined with the compositions provided herein are provided above and throughout this document. Advantageous vaccinating antigens that are suitable for inclusion in the compositions described herein include all or antigenic portions of bacteria, viruses, yeasts, fungi, protozoa and other microorganisms or pathogens of human, animal or plant origin and pollens and other allergens, including venoms (e.g., bee and wasp venoms), and asthma-inducing allergens such as house dust mite, cat or dog dander.

Additional advantageous vaccinating antigens include those provided hereinabove, as well as: viral antigens of influenza virus such as haemagglutinin protein (e.g., seasonal strains of inactivated influenza virus, recombinant HA antigen, and seasonal HI, H3 B or pandemic H5 antigen) and influenza nucleoprotein, antigens of the outer capsid proteins of rotavirus, antigens of human immunodeficiency virus (HIV) such as the gp120 protein of HIV, the surface proteins of respiratory syncytial virus (RSV), antigen E7 of human papilloma virus (HPV), Herpes simplex, Hepatitis A virus, Hepatitis B virus (e.g., HBsAg), surface proteins of Hepatitis C virus (HCV), inactivated Japanese encephalitis virus, surface proteins of Lyssavirus (causative of rabies); and antigens from microorganisms including but not limited to Shigella, Porphyromonas gingivalis (e.g., the proteinase and adhesin proteins), Helicobacter pylori (e.g., urease), Listeria monocytogenes, Mycobacterium tuberculosis (e.g., BCG), Mycobacterium avium (e.g., hsp65), Chlamydia trachomatis, Candida albicans (e.g., the outer membrane proteins of C. albicans), Streptococcus pneumoniae, Neisseria meningitidis (e.g., class 1 outer protein), Bacillus anthracis (causative of anthrax), Coxiella burnetii (causative of Q fever, but which can also induce long term protection against autoimmune diabetes (i.e., Type I diabetes)) and malaria-causing protozoa (such as Plasmodium falciparum and Plasmodium vivax).

Other advantageous antigens are cancer antigens (i.e., antigens associated with one or more cancers) such as: carcinoembryonic antigen (CEA), mucin-1 (MUC-1), epithelial tumor antigen (ETA), abnormal products of p53 and ras, and melanoma-associated antigen (MAGE). Other advantageous antigens include allergens for treating allergies by immunotherapy (e.g., pollen, house dust mites, grass pollens, peanut allergies, and the like).

Where the composition described herein includes a vaccinating antigen, the composition may also include an antigen-binding carrier material such as, for example, one or more metal salts or precipitates such as magnesium, calcium or aluminum phosphates, sulfates, hydroxides or hydrates thereof (e.g., aluminum hydroxide and/or aluminum sulfate) and/or one or more proteins, lipids, organic acids including sulfated or phosphorylated polysaccharides (e.g., heparin, dextran or cellulose derivatives), organic bases such as chitin (poly N-acetyl glucosamine) and deacetylated derivatives thereof, or basic cellulose derivatives, and/or other antigens. The antigen-binding carrier material may include poorly soluble particles of such materials (e.g., particles of aluminum hydroxide (alum) gel or a hydrated salt complex thereof). Advantageously, the antigen-binding carrier material does not tend to aggregate and/or may be treated to avoid aggregation. In some embodiments, the antigen-binding carrier material may be aluminum hydroxide (alum) gel, aluminum phosphate gel or calcium phosphate gel.

When the composition described herein includes a vaccinating antigen, the composition may also include pharmaceutically acceptable vehicle. Such compositions and preparations of the antigen should contain at least 0.1% of the active agent (e.g., the antigen of a (β-In or InAc composition described herein). The percentage of the active in the compositions and preparations may, of course, be varied and may conveniently be about 1% to about 60%, about 1% to about 10%, or about 2% to about 5%, of the weight of a given unit dosage form. The amount of active compound in such therapeutically useful compositions is such that an effective dosage level will be obtained.

When an antigen-binding carrier material is present in a composition, it may be present in a form that is intrinsically associated with the β-In or InAc, such as, for example, co-crystals with such materials. Co-crystals of a particulate form of β-In or InAc and an antigen-binding carrier material such as a metal salt may be prepared by, for example:
(a) preparing an inulin solution by heating β-In particles in water;
(b) adding to the solution an amount of one or more metal salts;
(c) recrystallizing the β-In from said solution to provide β-In co-crystalized with the metal salt; and
(d) isolating formed co-crystals of the β-In and one or more metal salts. The metal salt may be, for example, a phosphate, sulfate, hydroxide, or hydrate of magnesium, iron, calcium, aluminum, or the like.

The diameter of the particles of the β-In in combination with an antigen-binding carrier material such as a metal salt can be about 10 nm to 5 um, or about 150 nm to about 30 mm. Larger particles (e.g., greater than about 2 um in diameter) may be used in formulations such as gels. The particles of the β-In in combination with the antigen-binding carrier material may include a relative amount (by weight) of the inulin material to the antigen-binding carrier material in a ratio of about 1:20 to about 200:1.

In embodiments, a method of stimulating an immune response in a subject, or enhancing, eliciting, or decreasing or preventing an immune response in a subject, for the purposes of, for example, preventing, treating, or inhibiting an infectious disease, autoimmune disease, immunodeficiency disorder, neoplastic disease, degenerative or ageing disease is disclosed, wherein the method includes administering to a subject an effective amount of a formulation described herein. The formulations provided herein, when combined with antigens or other compounds provided hereinabove, are capable of creating immune capacity in a subject, creating immune recognition of antigen or other compound, as well as sensitizing the immune system of a subject to an antigen or other compound.

The term "effective amount" typically refers to a non-toxic but sufficient amount of the preparation/immunological composition to provide the desired effect. The exact amount required may vary from subject to subject depending on factors such as the species being treated, the age and general condition of the subject, the severity of the condition being treated, the particular preparation/immunological composition being administered, and the mode of administration etc. Thus, it is not always possible to specify an exact "effective amount". However, for any given case, an appropriate "effective amount" may be routinely determined by those of skill in the art.

Microparticles having particle diameters of about 2-5 microns, and nanoparticles having particle diameters of about 100-400 nm, may typically be prepared for as disclosed in the Examples herein. However, size ranges for the InAc microparticles as described herein may be about 1 um to about 30 um, or about 1.5 um to about 25 um. Size ranges for the InAc nanoparticles as described herein may be about 10 nm to about 1000 nm, 15 nm to about 950 nm, or 20 nm to about 900 nm.

The methods described herein may create, stimulate, illicit, enhance, augment, develop, boost, or improve an immune response in a subject by activation or modulation of mononuclear immune cell (e.g., monocyte, macrophage, and/or dendritic cell) function and/or the complement pathway in a human or non-human animal subject, for the purposes of inducing or modulating an immune response. The inducing or modulating of an immune response may be, for example, for the treatment, inhibition, or prevention of an infection by a bacterium, mycoplasma, fungus, virus, protozoan or other microorganism, or of an infestation by a worm or parasite or any of the above-mentioned antigens or pathogens, or to treat, inhibit, or prevent immuno-pathology induced by such an infection; the treatment or inhibition of an immune disorder such as an allergic or rheumatic disease, an autoimmune disease, an immunodeficiency disease, or neurological, dermatological, renal, respiratory or gastrointestinal disorders relating to dysfunction of the immune system; or the treatment or inhibition of a tumor or cancer cells, or the prevention or clearance of protein aggregation in neurodegenerative diseases such as Alzheimer's disease. As such, it is also to be understood that the invention extends to methods for treating, inhibiting, or preventing cancer in a subject, wherein the methods include administering to the subject an effective amount of a formulation described herein.

The following Examples are intended to illustrate the above invention and should not be construed as to narrow its scope. One skilled in the art will readily recognize that the Examples suggest many other ways in which the invention could be practiced. It should be understood that numerous variations and modifications may be made while remaining within the scope of the invention.

### EXAMPLES

### Example 1. Inulin Preparation.

1.1. Preparation of β-inulin (β-In). β-lnulin was prepared from raw inulin by an ethanol precipitation method. Commercially available raw inulin obtained from dahlia tubers (Thermo Fisher Scientific, USA) was suspended in ethanol and allowed to stand overnight at 4 °C. The next day, the precipitated β-inulin was separated after centrifugation and lyophilized. The dried β-inulin was then used for the further studies described below.
1.2. Synthesis of Inulin Acetate (InAc). Two grams of β-inulin was added to 15 mL of dimethyl formamide (DMF) to form a solution and was allowed to stir for complete solubilization of the β-inulin. Then 25 mL of acetic anhydride was added and the acetylation reaction was carried at 40 °C for 24 hours under nitrogen. Sodium acetate (0.1%, w/v) was used as a catalyst for the reaction. After 24 hours, InAc was precipitated in a large excess of cold water and was collected after filtration. InAc was washed two more times with water to remove any traces of unreacted β-inulin and was allowed to dry overnight. The prepared InAc was used for the further studies described below.

### Example 2. Preparation of Antigen-Loaded Microparticles.

2.1 Preparation of ovalbumin (ova)-loaded β-inulin Microparticles. The β-inulin microparticles were prepared by a single (w/o) emulsion nanoprecipitation technique. β-Inulin (100 mg) and ova (10 mg) were dissolved in 10 mL of 10 mM pH 7.4 phosphate buffer (aqueous phase). Fluorescein isothiocyanate (FITC) labeled ova was used instead of ova in microparticles preparation to evaluate loading and release profile of ova. The aqueous phase was added dropwise into 30 mL of light mineral oil containing 1% w/v of Tween-80 as a surfactant with continuous stirring (1000 rpm) to obtain a stable water in oil (w/o) emulsion. The emulsion was stirred for 4 hours and then 30 mL of acetone was added drop wise to precipitate the β-inulin microparticles. The emulsion was left stirring overnight and β-inulin microparticles were collected via centrifugation at 3000 g, 30 min at 4 °C. The pelleted ova-loaded β-inulin microparticles were then washed twice with n-hexane, kept at -80 °C for 1 hour and were lyophilized for 48 hours.
2.2. Preparation of ovalbumin-loaded InAc Microparticles. Ova-loaded InAc microparticles were prepared by a double (w/o/w) emulsion solvent evaporation technique. Briefly, 200 µL of 50 mg/mL ova solution was mixed with 50 µL of 10 mg/mL Pluronic F-68 solution (surfactant) in 10 mM phosphate buffer (pH 7.4) as an aqueous phase (W1). This aqueous phase was emulsified with 5 mL of dichloromethane (DCM) as an oil phase (O) containing 100 mg of InAc, resulting in the formation of primary (w/o) emulsion. This primary emulsion was then added dropwise into another aqueous (W2) phase (30 mL water) containing 0.5% w/v polyvinyl alcohol (PVA) as a surfactant, with continuous stirring (800 rpm) resulting in the formation of double (w/o/w) emulsion. The stirring was continued overnight for complete evaporation of the organic solvent. Then microparticles were collected via centrifugation at 50,000 g for 30 minutes at 4 °C. The supernatant was discarded and pelleted ova-loaded InAc microparticles were re-suspended in 100 mM citrate buffer pH 7.4, kept at -80 °C for 1 hour and then lyophilized (VirTis, Gardiner, NY) for 48 hours.
2.3. Preparation of ovalbumin-loaded InAc Nanoparticles. Ova loaded InAc nanoparticles were prepared by a double (w/o/w) emulsion solvent evaporation technique. Briefly, 200 µL of 50 mg/mL ova solution was mixed with 50 µL of 10 mg/mL Pluronic F-68 solution (surfactant) in 10 mM phosphate buffer (pH 7.4) as an aqueous phase. This aqueous phase was emulsified with 5 mL of dichloromethane (DCM) as an oil phase containing 100 mg of InAc using probe sonication for 20 s at 10 W (Sonics Vibracell, Newtown, CT), resulting in the formation of primary (w/o) emulsion. This primary emulsion was then emulsified with another aqueous phase (30 mL water) containing 3.0% w/v polyvinyl alcohol (PVA) as a surfactant using probe sonication for 120 s at 50 W, resulting in the formation of double (w/o/w) emulsion. The double emulsion was left for overnight stirring (800 rpm) for complete evaporation of the organic solvent. Ova-loaded InAc nanoparticles were then collected via centrifugation at 50,000 g for 30 min at 4 °C. The supernatant was discarded and the pelleted nanoparticles were re-suspended in 100 mM citrate buffer pH 7.4, kept at -80 °C for 1 hour and then lyophilized (VirTis, Gardiner, NY) for 48 hours.
2.4. Particle size and ovalbumin loading. Particles were dispersed in 10 mM citrate buffer (pH 7.4), which was previously filtered through a 0.22 µm pore size filter and suitably diluted for particle size measurement. Particle size was measured via dynamic light scattering method using a size and zeta potential Analyzer (Nicomp 360 ZLS, Santa Barbara, CA). Ova loaded β-inulin microparticles were 1.74 µm ± 0.14 in size and ova-loaded InAc microparticles were about 2 lam in size (Table 1).

To determine ova loading in β-inulin microparticles, a known quantity of FITC-ova loaded β-inulin microparticles was dissolved in 1% sodium dodecyl sulfate (SDS) solution. The ova content was determined by measuring fluorescence values of FITC-ova at excitation-490 nm and emission-530 nm. The ova concentration was calculated from the standard curve prepared using blank β-inulin microparticles spiked with known concentrations of FITC-ova. The ova loading reported as lug of ova present per mg of β-inulin microparticles (w/w). Ovalbumin loading was 75.9 ± 2.7 µg/mg with 75.3 % ± 4 of encapsulation efficiency (Table 1).

To determine ova loading in InAc micro or nano particles, a known quantity of ova loaded InAc micro- or nanoparticles was dissolved in acetone, and then precipitated protein was extracted with a 1% sodium dodecyl sulfate (SDS) solution. The ovalbumin content in the extract was measured via bicinchoninic acid (BCA) protein assay. The ova concentration was calculated from the standard curve prepared using blank InAc micro or nanoparticles dissolved in acetone and spiked with known concentrations of ova, which were further extracted in 1% SDS solution and analyzed via the BCA protein assay. The ova loading measured by this method reported as lug of ovalbumin present per mg of InAc micro or nano particles (w/w). Loading of ova was around 20.0 ± 5.4 µg/mg (Table 1).

**Table 1. Physicochemical Characterization of Ova-loaded Microparticles (MPs).**

| **Sample** | **Particles** | **Size** | **Ova loading (µg/mg)** |
|---|---|---|---|
| 1 | Ova-loaded β-Inulin MPs | 1.74 µm ± 0.14 | 75.9 ± 2.7 |
| 2 | Ova-loaded InAc MPs | 2.31 µm ± 0.32 | 20.0 + 5.4 |

| | | | |
|---|---|---|---|
| In Table 1, the data represents mean ± standard deviation (n=3). The ova loading refers to 1 µg of Ova present per mg of β-inulin or inulin acetate microparticles. | | | |

Several different sizes of particles were prepared. By varying and optimizing process and formulation parameters; such as sonication energy required, time of sonication, surfactant type, surfactant concentration, phase volume ratio, amount of antigen, and by the addition of other ingredients, nano- and micro-particles of different sizes, different loadings and different burst release amounts (percentage of antigen released in the first 30 min) were prepared. Nanoparticles of about 100 nm to 1000 nm, or about 220 nm to 800 nm, may be prepared using various modified conditions. The size and polydispersity of the particles may also be controlled by the modified conditions of microparticles or nanoparticles preparation.

InAc microparticles were made in the absence of sonication energy and in the presence of a low concentration of surfactants. The concentration of surfactant can depend on the type of surfactant and the size of the microparticles desired. With 0.5% of polyvinyl alcohol using approximately 800 r.p.m. stirring speed, the size of InAc microparticles obtained was about 2-3 µm. The size can be varied by changing the type of surfactant, the concentration of surfactant, phase volume ratio, solvent evaporation time and the stirring speed.

Particles of about 100 µm to about 200 µm may readily be prepared with slow stirring of the components (e.g., about 60 r.p.m.). The use of surfactants may help reduce or prevent aggregation of the particles formed. Accordingly, about 0.05% to about 3% of a surfactant by weight may be used to prepare the formulations. In some formulations, an amount of the surfactant, such as the PVA, may remain in the particles once formed. A cryoprotectant (e.g., mannitol or trehalose) may be used to for particles to be lyophilized.

By increasing the concentration of surfactant and by providing sonication energy to break the particles, InAc particles in the nanometer size (200-600 µm) may be generated. This range may vary depending on the amount of sonication energy provided, time of sonication, type and concentration of surfactant used, phase volume ratio, solvent evaporation time and the stirring speed of the formulation. Higher sonication energy (up to 50 watt was tested) for longer times (up to 5 minutes was tested) during second emulsion produced smaller particle sizes. Among several surfactants evaluated, use of PVA (3%) resulted in small particle, 260 ± 26 nm in diameter.

### Example 3. In-vitro Release Studies.

FITC-ova loaded β-inulin microparticles (10 mg) were dispersed in 1 mL of 100 mM phosphate buffer (pH 7.4) and incubated at 37 °C with 100 rpm shaking At predetermined time intervals tubes were taken out and centrifuged at 20,000 g for 10 min at 4 °C. A 50 µL aliquot of supernatant was taken for the measurement of released FITC-ova and replaced with an equal volume of fresh phosphate buffer. The released ova concentration in the supernatant was measured by fluorometric analysis. This in-vitro release study indicated that more than 90% of the ovalbumin was released within 16 hours (FIG. 6).

Ova-loaded InAc micro- or nanoparticles (10 mg) were dispersed in 1 mL of 100 mM phosphate buffer (pH 7.4) and incubated at 37 °C with 100 rpm shaking At predetermined time intervals tubes were taken and centrifuged at 20,000 g for 10 min at 4 °C. A 50 µL aliquot of supernatant was taken for measurement of released ova and replaced with an equal volume of fresh buffer. The ova concentration in the supernatant was measured via BCA assay. This in vitro release study showed that ova release was sustained for more than 20 days (FIG. 2). It took more than 20 days to release >90% of loaded ova compared to 16 hours in case of β-inulin.

### Example 4. Immunization Studies: Ova-loaded Particles as Vaccine Adjuvants and Delivery Systems.

Insoluble isoforms of inulin (gamma, delta and epsilon) have been tested as adjuvants for vaccines. However, inulin in its water soluble form, such as the β-polymorphic form, has never been shown to have an adjuvant/immune-potentiating effect. In the following studies, the immune-potentiating ability of ova-loaded β-inulin or InAc microparticles and nanoparticles were evaluated. The following immunization studies were performed using male Balb/C mice (n=4-5 per group, 6-8 weeks old).
4.1. Ova-loaded β-inulin microparticles immunization study. The mice were immunized via intradermal (i.d.) route with the following groups: i) ova (100 lug per mouse) in phosphate buffered saline (PBS); ii) physical mixture of ova (100 lug per mouse) and blank β-inulin microparticles in PBS; iii) 100 lug ova with 200 µg of Alum (aluminum hydroxide) in PBS; and iv) ova loaded β-inulin microparticles (equivalent to 100 µg ova) in PBS.
The vaccine formulations were administered at two different sites (50 µL at each site) to the shaved back skin of the mouse using a standard disposable 27 1/2-gauge syringe. A visible raised cutaneous swelling was regarded as evidence for successful i.d. administration. As per the immunization protocol, mice were vaccinated at "day 1" with the primary dose followed by a booster dose at "day 21". Blood samples were collected in serum gel tubes from the retro orbital plexus at 1^{st} and 3^{rd} weeks after primary and booster doses. Samples were centrifuged at 3000 g for 30 min and the sera were stored at -80 °C until further analysis.
The antibody titers (IgG-total, IgG-1 and IgG2a) produced by ova loaded β-inulin microparticles were significantly higher (p <0.05) than the alum adjuvanted ova group after booster immunization (FIG. 5). The β-inulin microparticles generated a greater IgG2a immune response than alum (FIG. 5C).
4.1.1. Uptake of antigen (ova) by dendritic cells. Dendritic cells (DC2.4) were incubated for 1 h with the FITC-ova in solution or loaded inside β-inulin microparticles at 37 °C. After 1 h incubation, cells were extensively washed, fixed in 4% (w/v) paraformaldehyde and analyzed using flow cytometry to determine the uptake of FITC-ova by DC2.4 cells (FIG. 3(A). Similar results were shown by fluorescence microscopy. DAPI shows nuclear staining of cells (FIG. 3(B)). The quantification of this data is represented in Table 2.

**Table 2. Flow cytometric analysis of antigen uptake by dendritic cells.**

| S. No. | Treatment Groups | Mean Fluorescence Intensity (counts) | % of green cells |
|---|---|---|---|
| 1 | No treatment | 4.60 ± 0.57 | 3.31 ± 1.61 |
| 2 | Ova in Solution | 13.18 ± 1.06 | 22.0 ± 2.68 |
| 3 | Ova loaded β-Inulin Microparticles | 324.16 ± 22.22* | 98.82 ± 0.71* |

| | | | |
|---|---|---|---|
| Dendritic cells were analyzed by flow cytometry after incubation with FITC-ova either in solution or inside β-inulin microparticles. Mean fluorescence intensity of FITC-ova in the green channel was represented as arbitrary fluorescence units. Cells were gated to remove auto-fluorescence observed in blank dendritic cells. Data represents mean ± standard deviation (n=3). * represents that the variance is significant (p<0.0001) compared to ova in the solution group. | | | |

4.1.2. Stimulation of Toll Like Receptors (TLRs) on antigen presenting cells (APCs). In addition to demonstrating Th1 and Th2 responses, various polymers were screened to identify candidates that stimulate TLRs on APCs. TLRs are a group of pattern recognition receptors (PRRs), when activated by pathogens through pathogen-associated molecular patterns (PAMPS), secrete/release several cytokines that drive the immune response toward Th1 and Th2 types. TLR activation (except for TLR3) requires an adaptor molecule called MyD88 or Mal to release cytokines such as TNF-α.
4.1.2.1. Inulin acetate (InAc) is a TLR-4 agonist. Wild type mouse macrophages cells and Mal/MyD88^{-/-} cells (1 x 10⁵ cells/well) were incubated with different formulations for 12 hours. Subsequently, the concentrations of TNF-α in the culture supernatant was measured by ELISA (see FIG. 4(A)). InAc stimulated the release of the cytokine TNF-α from My88 macrophages but failed to stimulate the release of TNF-α from macrophages that lack Mal and My88. HEK cells, which were stably transfected with TLR4 (1 x 10⁵ cells/well) receptor, were incubated with different formulations: media only, InAc microparticles (250 µg/ml), LPS (1 (µg/ml) and Zymosan (1 µg/ml). Cell supernatants were analyzed for IL-8 secretion by ELISA in triplicate wells after 16h of stimulation (see FIG. 4(B)).
As can be seen in from this data, InAc stimulates antigen presentation cells (APCs) to release cytokines (FIG. 4(A)). Further, it seems that InAc activates APCs through TLRs, especially through the activation of TLR4 receptors (FIG. 4(B)). InAc microparticles resulted in significantly enhanced TNF-α secretion from the regular (express MyD88 adaptor protein) macrophage cells. This secretion was abolished when TLR adaptor protein MyD88 was removed from the same cells (FIG. 4(A)). This clearly suggests that InAc activates immune cells with the help of TLRs. There are multiple TLR receptors. InAc was found to interact with TLR-4 specifically. (FIG. 4(B)). This is the first time that InAc has been shown to activate the innate immune system by interacting with TLR-4. Neither soluble β-inulin nor the γ-isoform could activate TLRs. Gamma inulin is known to activate the immune system through the alternative pathway of complement (ACP). The assay of ACP activation measures lysis of rabbit red blood cells (RBC) on activation of the APC present in normal human sera. As shown herein, neither β-inulin nor micro or nanoparticles of InAc could activate ACP. However as shown herein, γ-inulin and Zymosan activated ACP (see FIG. 16).
Based on these data, the β-inulin/InAc (micro or nanoparticles) may be working via a different mechanism than γ-inulin in functioning as adjuvants to activate an immune response. Further, by using the TLR-4 agonist (InAc), a particulate (nano/micro) vaccine delivery system has been identified and tested, where the system has the ability to stimulate an animal immune system (e.g., a mouse). A major finding is that the polymer used to make the delivery system is itself a TLR agonist. The delivery system does not need the addition of other PAMPS to enhance the immune response. For the immune system, InAc based particulate delivery systems are similar to pathogens in the way that they are particulate, consist of a polysaccharide based hydrophobic surface, may be used to encapsulate multiple antigens, and activate APCs (innate immune system) through TLRs by providing PAMP signaling.
4.2. The results of this study showed that ova-loaded β-inulin microparticles released the encapsulated antigen (ovalbumin) within 16 hours and generated significantly higher antibody titers (IgG-total and IgG-1) than the FDA approved alum.
In the following study, the water-insoluble β-inulin derivative inulin acetate (InAc) was used. The objective of this study was to further sustain the release of antigen for prolonged periods of time by encapsulating the antigen in InAc microparticles and to evaluate their immune-potentiating ability and use InAc as a novel TLR4 agonist to stimulate both humoral and cellular immune responses. A humoral response is needed to clear extracellular pathogens and a cellular response is needed for intracellular pathogens.
In the structure of InAc, the hydroxyl groups of β-inulin are substituted by acetyl groups. The disappearance of OH stretch band of β-inulin (-3326 cm⁻¹), and the appearance of a carbonyl band (C=0 ∼ 1743 cm⁻¹) in the FTIR spectrum of InAc confirms the synthesis of the acetate ester InAc from β-inulin (FIG. 6). In addition to the carbonyl band, inulin acetate is also characterized by the appearance of acetate C-0 band (∼1224 cm⁻¹) and -CH₃ band (-1369 cm⁻¹). Mice (n = 4-5 per group) were injected via intradermal (i.d.) route with the following groups: i) ova (100 lug per mouse) in phosphate buffered saline (PBS); ii) physical mixture of ova (100 µg per mouse) and blank InAc microparticles in PBS; iii) 100 µg ova with 200 µg alum (aluminum hydroxide) in PBS; and iv) ova loaded InAc microparticles (equivalent to 100 µg Ova) in PBS, on days 1 and 21 as primary and booster immunization. Sera were collected at 1 and 3 weeks after the primary and booster immunizations for analysis of IgG titers using indirect ELISA. The rest of the immunization protocol used was same as described in section 4.1.
Ova loaded InAc microparticles generated significantly higher (p < 0.001) antibody response than alum bound ova (Total IgG, 12-87 times; IgG1, 25-60 times; IgG2a, 7-1000 times). FIG. 7 depicts ova-specific IgG-Total, IgG-1 and IgG-2a antibody titers in immunized mice serum. Most interestingly, InAc microparticles generated both Th1 (IgG-2a) and Th2 (IgG-1) types of immune response, which is needed in modern vaccines. Encapsulation of ova in InAc microparticles resulted in enhanced immune response. When ova was simply co-injected with blank InAc microparticles or nanoparticles, no enhancement of immune response against ova was observed. However, prior art gamma-inulin or other water-insoluble forms of inulin, when co-injected as an adjuvant with an antigen, provide an enhanced immune response, but co-injection with beta-inulin or inulin acetate did not provide an enhanced immune response.
4.3. Ova-loaded InAc micro or nano particles immunization study: evaluation as vaccine adjuvants and delivery systems. This study was performed to evaluate the effect of particle size (micro vs. nano) of InAc particles and dose (100, 10 or 1 µg) of an antigen (ovalbumin) used on generation of an immune response. Formulations with different particle sizes and different ova loading were prepared. Optimization studies using a factorial design approach where the following formulation and process parameters were varied were performed.
1) Amount of antigen (ova) used during first emulsification step of InAc micro or nanoparticles preparation. Ova loading (µg/mg) was increased from 0.5 µg /mg to 50 µg/mg, by increasing the amount of ova (500 µg to 20 mg per 100 mg of polymer) used during particles preparation. However, burst release also increased at higher (50 µg/mg) ova loading.
2) By increasing the second aqueous phase volume to 45 mL in the preparation of the InAc microparticles and nanoparticles, the burst release was restricted to about 20%.
3) To reduce the size of InAc particles, sonication energy was used. The process parameters, including time and energy of sonication, were optimized to provide a nanometer size range of the particles (approximately 100-600 nm) with desired loading (e.g., 10 µg/mg to about 100 µg/mg for InAc particles and about 10 µg/mg to about 500 µg/mg for β-inulin particles) and restricted burst release (less than about 30%, or less than about 20%, in the first 30 minutes post-administration).
By optimizing these three parameters, the desired formulations with various sizes (approximately 100 nm-1000 nm for nanoparticles; and approximately 1 µm-30 µm for microparticles) and loading can be successfully prepared.
To study the effect of particle size on stimulated immune response, mice were immunized with ova-loaded InAc microparticles (approximate size: 2 µm) or nanoparticles (approximate size: 250 nm) with varied doses of ova. The mice were immunized via subcutaneous (s.c.) route with the following groups: i) ova (100, 10 or 1 µg per mouse) in PBS; ii) ova (100, 10 or 1 µg per mouse) with 100 µm of CFA emulsion; and iii) ova loaded InAc micro or nano particles (equivalent to 100, 10 or 1 µg of ova) in PBS. The rest of the immunization protocol was same as described in section 4.1.
FIG. 11 shows the effect of size of inulin acetate particles on generation of ova-specific IgG-total titers in immunized mice serum. Mice (n = 4-5 per group) were injected subcutaneously with ova (100, 10 or 1 µg) alone or along with CFA or loaded in InAc micro or nanoparticles on days 1 and 21 as primary and booster immunization. Sera were collected at 1 and 3 weeks after the primary and booster immunizations for analysis of IgG-total titers using indirect ELISA. CFA was used as a positive control (strongest adjuvant).
FIG. 12 shows the effect of size of inulin acetate particles on generation of serum ova specific IgG-1 titers in immunized mice serum. Mice (n = 4-5 per group) were injected subcutaneously with ova (100, 10 or 1 µg) alone or along with CFA or loaded in InAc micro or nanoparticles on days 1 and 21. Sera were collected at 1 and 3 weeks after the primary and booster immunizations for analysis of IgG-1 titers using indirect ELISA.
FIG. 13 shows the effect of size of inulin acetate particles on generation of ova-specific IgG-2a titers in immunized mice serum. Mice (n = 4-5 per group) were injected subcutaneously with ova (100, 10 or 1 µg) alone or along with CFA or loaded in InAc micro or nanoparticles on days 1 and 21. Sera were collected at 1 and 3 weeks after the primary and booster immunizations for analysis of IgG-2a titers using indirect ELISA.
InAc microparticles produced higher antibody titers than nanoparticles at 100 and 10 lug doses of ova and the antibody titers were even higher than the positive control CFA adjuvanted group. However, nanoparticles produced higher antibody titers than microparticles at 1 µg dose of an antigen (FIGs. 11, 12 and 13). This data indicates that InAc microparticles are more potent than CFA in generation of immune response at 10 and 100 µg dose of an antigen.
To study the effect of dose of an antigen, mice were immunized with 100, 10 or 1 µg ovalbumin loaded in InAc micro or nanoparticles. FIG. 14 shows the effect of amount of an antigen loaded in InAc microparticles on generation of ova-specific IgG titers in immunized mice serum. Mice (n = 4-5 per group) were injected subcutaneously with ova (100, 10 or 1 µg) alone or along with CFA or loaded in InAc microparticles on days 1 and 21. Sera were collected at 1 and 3 weeks after the primary and booster immunizations for analysis of IgG-total, IgG-1 and IgG-2a titers using indirect ELISA.
FIG. 15 shows the effect of amount of an antigen loaded in InAc nanoparticles on generation of ova-specific IgG titers in immunized mice serum. Mice (n = 4-5 per group) were injected subcutaneously with ova (100, 10 or 1 µg) alone or along with CFA or loaded in InAc nanoparticles on days 1 and 21. Sera were collected at 1 and 3 weeks after the primary and booster immunizations for analysis IgG-total, IgG-1 and IgG-2a titers using indirect ELISA.
Ova-loaded InAc microparticles showed that the immune response depends on the dose of ova with highest antibody titers at 100 µg of an antigen followed by 10 µg and 1 µg dose (FIG. 14). Ova-loaded InAc nanoparticles also generated stronger antibody titers at 100 µg of an antigen as compared to 10 µg and µg of an antigen, but there was no significant difference found in antibody titers at 10 µg and 1 µg of an antigen dose (FIG. 15).
4.4. Detection of anti-ova antibodies using Enzyme-Linked Immunosorbent Assay (ELISA). Sera from the immunized mice were tested for the presence of antibodies (Total IgG, IgG1 and IgG2a) generated against ova by an Indirect-ELISA assay method. Briefly, 96-well ELISA plates were coated with ova (1 µg/well) in pH 9.6 carbonate buffer and incubated overnight at 4 °C. The plates were washed with wash solution (50 mM Tris, 0.14 M NaCl, 0.05% Tween 20, pH 8), and then blocked with 200 µL of blocking solution (50 mM Tris, 0.14M NaCl, 1% BSA, pH 8) for 30 min at room temperature (-23 °C). After washing the plates with wash buffer, the wells in plates were incubated with different dilutions of test sera (100 µL) for 1 hour at room temperature. The plates were washed and subsequently incubated with 100 µL of peroxidase-conjugated goat anti-mouse antibodies for 1 hour at room temperature. After incubation, the plates were washed and incubated with 100 µL of 3,3',5,5'-tetramethylbenzidine (TMB) substrate solution for 5 min at room temperature for color development. The reaction was stopped using 2M H₂SO₄ and optical density (OD) was measured at 450 nm. Results were expressed as serum immunoglobulin G (IgG) titers, which are defined as the reciprocal end serum dilution at which the OD is more than average OD plus two standard deviations of the PBS control.
4.5. Splenocyte proliferation assay. Elicitation of memory responses is essential if vaccines are to confer long-lasting protection. To evaluate generation of a memory response, three weeks after the booster immunization, splenocytes were prepared from the spleens of mice.
On day 21 following the booster immunization, mice were sacrificed and the spleens were removed. Single cell suspension of the splenocytes was prepared in complete RPMI media (RPMI 1640 medium supplemented with 10% heat inactivated fetal bovine serum (FBS), 1% penicillin/streptomycin, 1 mM sodium pyruvate and 50 µM 2-mercaptoethanol). The cell suspension was centrifuged at 700 g at 25 °C for 5 min. After discarding the supernatant, RBCs were lysed using 100 mM NH₄Cl. The splenocytes were resuspended in complete RPMI media and cells were counted by trypan blue exclusion using a Cellometer (Nexcelom Bioscience, Lawrence, MA). Splenocytes (10⁶ cells/well) were seeded into 96 well plates and incubated with 200 µL of complete RPMI 1640 media only, containing ovalbumin (100 µg/mL) or Concanavalin A (2.5 µg/mL). After 72 hours of incubation, the supernatant was removed and saved for cytokine analysis. The cells were incubated with 50 µL of 3-(4,5-dimethylthiazol-2-yl)-2,5-diphenyl-tetrazolium bromide (MTT) solution (0.5 mg/mL) for 4 hours. At the end the plates were incubated with 150 µL of dimethyl sulfoxide (DMSO) at 37 °C for 10 minutes. Then plates were read at 540 nm using UV-Vis spectrophotometer. Stimulation index (SI) was calculated by dividing the absorbance values of cells treated with Concanavalin A or ova with the absorbance values of RPMI treated cells.
The data indicate that memory T cells that recognize ova during consequent exposures were generated in a significantly higher number in ova-loaded InAc microparticles treated mice (FIG. 8). The mitogen Concanavalin A (ConA) served as a positive control and it enhanced the stimulation index (S.I.) non-specifically in all the groups.
4.6. Cytokine Analysis: Th1 (IFN-g and IL-2) and Th2 (IL-4 and IL-10) cytokine measurements. To evaluate whether ova-loaded InAc microparticles induced preferentially humoral (Th2) or cell-mediated immune responses (Th1), the presence of secreted Th1 cytokines (IL-2 and IFNγ) and Th2 cytokines (IL-4 and IL-10) in splenocyte culture supernatants were examined after re-stimulation with ova.
Splenocytes were prepared from mice immunized with Ova alone, Ova with Alum, or Ova loaded InAc microparticles and were cultured for 72 hours in the presence of Ova (100 µ/mL). After 72 hours, supernatant from different treatment groups were collected and concentration of different cytokines were measured using sandwich ELISA. See FIG. 9. The asterisk (*) indicates the values are significantly higher compared to ova immunized group (P <0.001) using the student t-test.
In support of the antibody response data (high levels of both IgG-1 and IgG-2a), high levels of both (Thl and Th2) types of cytokines were observed in the splenocyte supernatants of mice immunized with the ova-loaded InAc microparticles (FIG. 9).
4.7. Delayed-Type Hypersensitivity (DTH) Response.
DTH responses were measured by injecting 5 µg of ova in the left footpad and equal volume of PBS in the right footpad of each immunized mouse. The degree of footpad swelling after 24 hours of the treatments was measured by subtracting thickness of right footpad from left footpad. The data in FIG. 10 represents the mean degree of swelling + standard deviation from 3-4 immunized mice of each group. The asterisk (*) indicates values that are statistically significant compared to the ova immunized group (P < 0.001) using the student t-test.
Analysis of the DTH response further confirmed the generation of a Th1 type of immune response. Mice immunized with ova loaded InAc microparticles generated a significantly higher (p < 0.001) DTH response than ova immunized mice (FIG. 10). These results further support a Th1 type of immune response induced by ova-loaded InAc microparticles.
4.8. Alternative pathway of complement (APC) activation assay. The insoluble polymorph of inulin (gamma-inulin) has been shown to act as a vaccine adjuvant by activating APC (Silva et al., Immunol. Cell Biol. (2004) 82, 611-616). However, β-inulin has never been shown to act as a vaccine adjuvant/immune-potentiator. The potential of β-inulin particles, InAc microparticles and InAc nanoparticles to activate APC was evaluated as described below.

Human serum causes the lysis of rabbit RBCs, and therefore, human sera and rabbit red blood cells (RBC's) were used for an APC activation assay. Rabbit RBC's were washed using APC buffer (Gelatin Veronal Buffer (GVB buffer) + 5 mM ethylene glycol tetraacetic acid (EGTA)). For the APC activation assay, 100 µL of human serum containing 1 mg/mL of inulin, β-inulin, gamma-inulin, Zymosan (positive control for APC activation), InAc microparticles and InAc nanoparticles were diluted with 400 µL of GVB buffer, incubated at 37 °C for 30 min and centrifuged. The supernatant was incubated with 500 µL of RBC's (1x10⁸ cells/mL) at 37 °C for 45 min, and then O.D. value at 700 nm was observed. Percent RBC lysis was calculated by considering the RBC lysis with untreated human serum as 100 %. The data was reported as % 10 lysed RBC's. Diluted human serum in GVB buffer upon incubation with RBC's resulted in 100% RBC's lysis. Zymosan activates APC and hence inhibited the rabbit RBC's lysis.

The assay of APC activation measures lysis of rabbit red blood cells (RBC) on activation of the alternative pathway of complement present in normal human serum. The data shown in FIG. 16 indicates that β-inulin, InAc microparticles and InAc nanoparticles did not activate APC, unlike gamma-inulin and Zymosan. Accordingly, the β-inulin, InAc microparticles and InAc nanoparticles operate by different mechanisms than gamma-inulin.

### Example 5. Safety

Mouse tissue was analyzed by hematoxylin and eosin (H&E) staining after immunization with various adjuvant systems (InAc nanoparticles, InAc microparticles and complete Freud's adjuvant (CFA)). Images were developed for the sites of injection 21 days after vaccine administration. Subcutaneous injection of InAc microparticles (2-4 microns in diameter) formed a local depot at the site of injection similar to alum or CFA and sustained the release of antigen for > 20 days. However, InAc nanoparticles (mean diameter 200-300 nm) were cleared from the site of injection and were probably targeted to the lymphatic system. In either case, there was no inflammation, tissue damage or abscess formation observed (FIG. 17(A)).

Strong adjuvant activity is often associated with toxicity. For example, the FDA approved vaccine adjuvant, alum, also causes pain, inflammation, lymphadenopathy, necrosis, and granuloma at the injection site. The safety of the vaccine formulation was analyzed as by determining the gross structural damage at the injection site in mouse skin.

A clear difference between CFA treatment and InAc microparticles may be seen. Ova loaded InAc microparticle treated sites have very high numbers of immune infiltrating cells and little to no tissue damage at the injection site as compared to CFA treatment. As expected, CFA injection resulted in severe tissue necrosis (shown by black arrows) at the injection site. In contrast, InAc microparticles did not cause any major toxicity at the injection site. The number of immune infiltrating cells (shown by white arrows) at the injection sites was found to be greater with the InAc microparticles compared to the CFA treatment. Again, these data clearly suggest that InAc microparticles are not only potent vaccine adjuvants, but also show a good safety profile.

### Example 6. Pharmaceutical Dosage Forms

The following formulations illustrate representative pharmaceutical dosage forms that may be used for the therapeutic or prophylactic administration of a β-inulin or InAc composition described herein, a β-inulin or InAc composition specifically disclosed herein, or a β-inulin or InAc composition in combination with other components described herein (hereinafter referred to as 'Composition X'):

| (i) Tablet 1 | mg/tablet |
|---|---|
| 'Composition X' | 100.0 |
| Lactose | 77.5 |
| Povidone | 15.0 |
| Croscarmellose sodium | 12.0 |
| Microcrystalline cellulose | 92.5 |
| Magnesium stearate | 3.0 |
| | 300.0 |

| (ii) Tablet 2 | mg/tablet |
|---|---|
| 'Composition X' | 20.0 |
| Microcrystalline cellulose | 410.0 |
| Starch | 50.0 |
| Sodium starch glycolate | 15.0 |
| Magnesium stearate | 5.0 |
| | 500.0 |

| (iii) Capsule | mg/capsule |
|---|---|
| 'Composition X' | 10.0 |
| Colloidal silicon dioxide | 1.5 |
| Lactose | 465.5 |
| Pregelatinized starch | 120.0 |
| Magnesium stearate | 3.0 |
| | 600.0 |

| (iv) Injection 1 (1 mg/mL) | g/mL |
|---|---|
| 'Composition X' (free acid form) | 1.0 |
| Dibasic sodium phosphate | 2.0 |
| Monobasic sodium phosphate | 0.7 |
| Sodium chloride | 4.5 |
| 1.0 N Sodium hydroxide solution (pH adjustment to 7.0-7.5) | q.s. |
| Water for injection | q.s. ad 1 mL |

| (v) Injection 2 (10 mg/mL) | mg/mL |
|---|---|
| Composition X' (free acid form) | 10.0 |
| Monobasic sodium phosphate | 0.3 |
| Dibasic sodium phosphate | 1.1 |
| Polyethylene glycol 400 | 200.0 |
| 0.1 N Sodium hydroxide solution (pH adjustment to 7.0-7.5) | q.s. |
| Water for injection | q.s. ad 1 mL |

| (vi) Aerosol | mg/can |
|---|---|
| Composition X' | 20 |
| Oleic acid | 10 |
| Trichloromonofluoromethane | 5,000 |
| Dichlorodifluoromethane | 10,000 |
| Dichlorotetrafluoroethane | 5,000 |

| (vii) Topical Gel 1 | wt.% |
|---|---|
| 'Composition X' | 5% |
| Carbomer 934 | 1.25% |
| Triethanolamine (pH adjustment to 5-7) | q.s. |
| Methyl paraben | 0.2% |
| Purified water | q.s. to 100g |

| (viii)Topical Gel 2 | wt.% |
|---|---|
| 'Composition X' | 5% |
| Methylcellulose | 2% |
| Methyl paraben | 0.2% |
| Propyl paraben | 0.02% |
| Purified water | q.s. to 100g |

| (ix)Topical Ointment | wt.% |
|---|---|
| 'Composition X' | 5% |
| Propylene glycol | 1% |
| Anhydrous ointment base | 40% |
| Polysorbate 80 | 2% |
| Methyl paraben | 0.2% |
| Purified water | q.s. to 100g |

| (x) Topical Cream 1 | wt.% |
|---|---|
| 'Composition X' | 5% |
| White bees wax | 10% |
| Liquid paraffin | 30% |
| Benzyl alcohol | 5% |
| Purified water | q.s. to 100g |

| (xi) Topical Cream 2 | wt.% |
|---|---|
| 'Composition X' | 5% |
| Stearic acid | 10% |
| Glyceryl monostearate | 3% |
| Polyoxyethylene stearyl ether | 3% |
| Sorbitol | 5% |
| Isopropyl palmitate | 2% |
| Methyl paraben | 0.2% |
| Purified water | q.s. to 100g |

These formulations may be prepared by conventional procedures well known in the pharmaceutical art. It will be appreciated that the above pharmaceutical compositions may be varied according to well-known pharmaceutical techniques to accommodate differing amounts and types of active ingredient 'Composition X'. Aerosol formulation (vi) may be used in conjunction with a standard, metered dose aerosol dispenser. Additionally, the specific ingredients and proportions are for illustrative purposes. Ingredients may be exchanged for suitable equivalents and proportions may be varied, according to the desired properties of the dosage form of interest.

While specific embodiments have been described above with reference to the disclosed embodiments and examples, such embodiments are only illustrative and do not limit the scope of the invention. Changes and modifications can be made in accordance with ordinary skill in the art without departing from the invention in its broader aspects as defined in the following claims.

All publications, patents, and patent documents are incorporated by reference herein, as though individually incorporated by reference.

## Claims

1. A composition comprising microparticles or nanoparticles of β-inulin or inulin acetate (InAc) and an active agent.

2. The composition according to claim 1, wherein the composition comprises microparticles having diameters of about 1 µm to about 30 µm.

3. The composition according to claim 1, wherein the composition comprises nanoparticles having diameters of about 10 nm to about 1000 nm.

4. The composition according to any of the claims 1 to 3, wherein the β-inulin or InAc has a molecular weight of about 4 kDa to about 16 kDa.

5. The composition according to any of the claims 1 to 4, further comprising one or more immune modulators, wherein the one or more immune modulators are lymphokines, cytokines, thymocyte stimulators, monocyte or macrophage stimulators, endotoxins, or a combination thereof, and wherein the active agent comprises an antigen, DNA, RNA, an antigenic peptide, or antigenic sequence, an immunogen, or an immunoglobulin or wherein the active agent comprises a lysate of a bacterial cell, cancer cell, fragments of a viral pathogen or other biological components associated with a pathogen or disease.

6. The composition according to any of the claims 1 to 5, wherein the composition further comprises an effective amount of a second active agent.

7. The composition according to any of the claims 1 to 6, wherein the composition stimulates an immune response in vivo or in situ and the immune response comprises Th1 (IgG-2a) or Th2 (IgG-1) types of immune response, or a combination thereof

8. The composition according to any of the claims 1 to 7, wherein the composition is a vaccine comprising inulin acetate, and wherein the degree of acetylation on the inulin acetate is about 0.1% to 100%.

9. The composition according to any of the claims 1 to 8, wherein the β-inulin is an inulin derivative selected from the group consisting of esterified inulin, etherified inulin, dialdehyde inulin, inulin carbamate, inulin carbonate, oxidized or reduced forms of inulin, and inulin phosphates.

10. A composition comprising microparticles or nanoparticles of β-inulin or inulin acetate (InAc) and an active agent according to any of the claims 1 to 9 for use in the treatment of an infectious disease, autoimmune disease, genetic disease, allergy, diabetes, immunodeficiency disorder, neoplastic disease, degenerative or ageing disease.

11. The composition according to claim 10, wherein the composition induces an immunogenic response in a subject via TLRs.

12. A kit comprising:
a) a composition comprising microparticles or nanoparticles of β-inulin or inulin acetate (InAc) and an active agent according to any of the claims 1 to 9;
b) a container;
c) one or more buffers,
d) instructions for associating an active agent with said composition; and
e) a label.

13. The kit according to claim 12, wherein the active agent is selected from the group consisting of an antigen, DNA, RNA, an antigenic peptide, or antigenic sequence, an immunogen, or an immunoglobulin.

14. The kit according to claims 12 or 13, further comprising instructions for producing antisera in an animal or human.

15. The kit according to any of the claims 12 to 14, wherein the β-inulin is an inulin derivative selected from the group consisting of esterified inulin, etherified inulin, dialdehyde inulin, inulin carbamate, inulin carbonate, oxidized or reduced forms of inulin, and inulin phosphates.

16. The kit according to any of the claims 12 to 15, further comprising one or more immune modulators, wherein the one or more immune modulators are lymphokines, cytokines, thymocyte stimulators, monocyte or macrophage stimulators, endotoxins, or a combination thereof, and wherein the active agent comprises an antigen, DNA, RNA, an antigenic peptide, or antigenic sequence, an immunogen, or an immunoglobulin or wherein the active agent comprises a lysate of a bacterial cell, cancer cell, fragments of a viral pathogen or other biological components associated with a pathogen or disease.

17. A method for stimulating, inducing and/or enhancing a immunogenic response in a subject using a composition comprising microparticles or nanoparticles of β-inulin or inulin acetate (InAc) and an active agent according to any of the claims 1 to 9.

18. A method for producing antibodies for stimulating immune cells in a subject using a composition comprising microparticles or nanoparticles of β-inulin or inulin acetate (InAc) and an active agent according to any of the claims 1 to 9.

19. A method for producing antisera in a subject using a composition comprising microparticles or nanoparticles of β-inulin or inulin acetate (InAc) and an active agent according to any of the claims 1 to 9.
